# EUROPEAN PATENT APPLICATION

(11) **EP 4 306 077 A1**
(43) Date of publication of application: **17.01.2024**
(21) Application number: 22185166.0
(22) Date of filing: 15.07.2022
(51) Int. Cl.: A61F 2/14

(54) **A METHOD AND A SYSTEM OF DETERMINING SHAPE AND APPEARANCE INFORMATION OF AN OCULAR PROSTHESIS FOR A PATIENT, A COMPUTER PROGRAM PRODUCT AND A CONFORMER**

(71) Applicant: Fraunhofer-Gesellschaft zur Förderung der angewandten Forschung e.V., 80686 München (DE)
(72) Inventor: Reinhard, Johann, 64293 Darmstadt (DE); Urban, Dr. Philipp, 64342 Seeheim (DE); Brunton, PhD Alan, 38000 Grenoble (FR)
(74) Representative: Ramrath, Lukas

(57) **Abstract**

Determination of the shape of the ocular prosthesis comprising the steps of - determining the shape based on a shape model being determined based on the shapes of existing prostheses or - determining the shape by generating shape information of an existing patient-specific prosthesis and transforming it into a uniform shape representation,
and/or the generation of appearance information comprises performing at least one of
- an inhomogeneous illumination correction and - a color characterization being performed using the same or similar viewing conditions as a color characterization of a device used for the manufacturing of the ocular prosthesis based on the appearance information.

## Description

The invention relates to a method and a system of determining shape and appearance information of an ocular prosthesis for a patient, a computer program product and a conformer.

Ocular prostheses may be worn by anophthalmic patients (patients who have lost an eye, because of a disease, congenital defect or trauma) or microphthalmic patients (patients with an eve that is abnormally small, due to a birth defect).

GB 2589698 A1 relates to ocular prostheses, prosthetic eyes, and methods of manufacturing ocular prostheses. Figure 1 of said document schematically illustrates an example of a prosthetic eye 1 in an anophthalmic eye socket 2. When the patient's eye is removed, the optic nerve 3 is severed, and the eyeball is detached from the muscles 4 which control its movement. After the eyeball is taken out of the socket 2, the patient is then fitted with an orbital implant 5, to which the muscles 4 are reattached. The implant 5 is covered with the conjunctiva skin layer 6, and when the swelling from the surgery dies down, an ocular prosthesis 7 is provided. The ocular prosthesis 7 should match the aesthetic appearance of the real eye, and is removable, for regular cleaning. For a microphthalmic patient, the ocular prosthesis 7 is worn with the existing eyeball, rather than the orbital implant 5. In either case, the ocular prosthesis 7 is custom made for both size and appearance. The prosthesis 7 should form a loose friction fit with the implant 5 or a microphthalmic eye. Thus, when the implant 5 or microphthalmic eye is moved by the muscles 4, the prosthesis 7 should move in a corresponding manner. However, the friction fit should also provide for sufficient oxygenation of the eye socket. Currently, the process for making an ocular prosthesis 7 involves making an impression of the anophthalmic socket or microphthalmic eye and surrounding tissue using an impression material. The body of the prosthesis 7 is then moulded by hand using the impression. A separate disc forming the iris is made, and attached to the moulded body. A transparent cornea is then over moulded on top of the body. The shape of the rear surface of the ocular prosthesis 7 is based on the impression. The rest of the shape of the prosthesis 7 is determined based on standard parts. Both the iris and the sclera are hand painted whilst the patient is present in clinic to provide the desired appearance. Alternatively, an image can be used as a reference for hand painting.

The process of taking the impression can be uncomfortable and difficult for both the patient and the ocularist taking the impression. In addition, because of these difficulties in the process of taking the impression, the impression may be inaccurate. This may cause a poor fit of the prosthesis 7 to the implant 5 or microphthalmic eye, such that the prosthesis 7 requires modification or, is uncomfortable and /or does not move as it should.

Furthermore, the process from taking the impression to completing the eye can take several weeks or more. Younger patients may need to repeat the process on a regular basis to be fitted for new prostheses as they grow.

In other words, most artificial eyes are produced in a completely manual labor process, where the ocularist uses a paste that is pressed into the eye socket to form a so-called alginate impression. This wax model is used to sculpt the shape of the artificial eye. To replicate the shape the ocularist usually paints the iris on disks with a fixed diameter using a fine brush, similarly the staining is painted while the veins are often simulated with yarn. For painting, often a (non-color-calibrated) picture of the companion eye with a non-color-calibrated display / print is used as a reference for the ocularist. There are also some manufacturers that produce pre-made iris disks in all colors using
different printing techniques. Overall, this process is very slow and labor intensive with varying quality based on the ocularists skills.

Recently some researchers have experimented using full-color 3D printing technology to create artificial eye prostheses. However, in all those cases the model was designed manually using CAD software, requiring the designer to be a domain expert.

US 2018/0012401 A1 relates to generating a parametric eye model but not to the generation of an ocular prosthesis.

There is the technical problem of providing a method and a system of determining shape and appearance information of an ocular prosthesis for a patient, a computer program product and a conformer which allow an accurate shape and appearance determination, in particular for manufacturing an ocular prosthesis with a good wearing comfort for the patient and an accurate appearance reproduction.

The solution to said technical problem is provided by the subject-matter with the features of the independent claims. Advantageous embodiments are provided by the subject-matter with the features of the remaining sub claims.

The invention addresses the problem of creating an ocular prosthesis from data, in particular image and scan data, of an eye and the patients anophthalmic socket (missing eye) or microphthalmic eye surface (defective small eye) in such a way that the prosthesis matches in shape a patient's eye socket or surface and in appearance the patient's companion eye or enucleated eye.

A method of determining shape and appearance information of an ocular prosthesis for a patient, in particular for the manufacturing of the ocular prosthesis, is proposed. The method comprises the steps of:
- generating shape information for the ocular prosthesis,
- determining the shape of the ocular prosthesis depending on said shape information,
- generating appearance information for the ocular prosthesis by capturing an image of a patient's eye,
- fusing the shape and the appearance information,

The generation of shape information can comprise the scanning (which can also be referred to as imaging) of an eye socket or of an existing ocular prosthesis and therefore, said shape information can also be referred to as measured shape information. An existing prosthesis can be a patient-specific prosthesis which is already adapted in shape to the patient's eye socket. The scanning can be performed by suitable means, i.e. a scanning device which can also be referred to as shape scanning device. Scanning an eye socket is preferably performed by an OCT (optical coherence tomography) scanning device which can provide two- or three-dimensional, i.e. volumetric, image data of the eye socket, in particular of an anterior surface of an orbital implant, in particular if covered by the conjunctiva. Such an OCT scanning is known in the art. It is, however, possible that other means, such as a laser scanning device can be used in order to provide the shape information. Further, the target tissue surface, i.e. the surface to which the prosthesis should be attached, can be extracted from the scan, in particular the volumetric scan.

It is also possible that the shape scanning device can (additionally) provide mesh information, e.g. three-dimensional information of the imaged eye. These mesh information can be provided by processing a raw signal of the shape scanning device. Such mesh information can be used to identify predetermined regions of the imaged eye such as the sclera region, cornea region or the iris region. As a result of such an identification, region-labelled mesh data can be provided, e.g. a cornea mesh, an iris mesh or a sclera mesh. The correspondence of such mesh information and the scan data can be predetermined, e.g. by a calibration. In other words, the mesh information can be co-registered to the scan data.

For generating the shape information by scanning the eye socket, the surface on which the prosthesis is to be fitted can be captured/measured. The shape information can in particular encode a spatial course of the anterior surface or a section thereof or of a line within said surface. The shape information can be generated relative to a reference coordinate system which will be explained later. These measured shape information provide in particular information for the shape of a rear or posterior surface of the prosthesis. Generating such shape information can be performed while an orbital implant is in place. It is further preferred to generate shape information while a temporary prosthesis (which can also be referred to as conformer) is in place which helps to hold the patient's eyelids open and to allow to use known OCT techniques.

The shape of the ocular prosthesis to be manufactured (also referred to as resulting shape) which is determined based on said shape information can be determined as a vertex-based representation of the shape, in particular as a point cloud in a 3D space. The shape information can also be determined as a mesh, e.g. with faces connecting points of the point cloud. Vertices or points of said representation can be determined as region-labelled vertices/points which means that such a vertex or point can be assigned to a region of the eye such as the sclera, the iris, the pupil etc.

Generating appearance information for the ocular prosthesis by capturing an image of a patient's eye can involve the application of imaging techniques in order to reproduce the visual appearance of the companion eye.

If the appearance information is determined as texture information, fusing the shape and the appearance information can include mapping the texture information to the shape, e.g. by the well-known spherical or cone-like UV-mapping. It is possible to identify corresponding regions in the shape and the appearance information and map the region-specific appearance information on the corresponding region in the shape information. As an example, an iris region can be extracted from the appearance information, e.g. by segmentation, and then be mapped on an iris geometry in the determined shape. In addition to color information, the texture information can also comprise transparency information and/or displacement information.

The determination of the shape of the ocular prosthesis according to a first aspect of the invention comprises determining the shape based on a shape model, wherein said shape model is determined based on the shapes of existing prostheses. Such a determination is in particular performed if the measured shape information is generated by scanning the eye socket. In other words, existing prostheses are used to determine a shape model, in particular a so-called statistical shape model. This shape model and the measured shape information, e.g. of the eye socket, are then used to determine the resulting shape of the prosthesis. This can be done by matching the measured shape information to the shape model.

The shape model can be established by using shape modelling algorithms known in the art. As a non-limiting example, the shape model can be established by machine learning algorithms. Using training data, in particular data representing the shapes of existing prostheses, preferably shapes according to an uniform shape representation, such an algorithm is used to provide a shape model. In this case, modelling can be performed using an autoencoder or a variational autoencoder (VAE) or a generative adversarial network (GAN). Training the neural network can comprise determining a preset number of latent variables such that a difference between input data to an encoding part of the network and output data of a decoding part of the network is minimized. Input data can be provided by the shape data of the existing prostheses, in particular in the uniform representation, wherein the output data is also shape data, in particular in the uniform representation. After the network has been trained, the latent variables and the decoding part can be used to provide the shape model, wherein the latent variables can provide model parameters which can be varied in order to vary the shape (within the limits of the shape model).

The shape model can also be established using principal component analysis (PCA). In this case, 3D points, in particular surface points, of existing prostheses in a common coordinate system can provide a set of points for which the principal components are determined. Once the principal components are determined, a (model) shape can then be represented as a linear combination of principal components or a predetermined set of principal components, e.g. the 1^{st} to the n-th principal component, wherein n can be a predetermined number. Experiments have shown that n=22 provides desirable results. In this case, a model parameter can be the weight assigned to one of the principal components in the linear combination.

The shape model can be determined in a model space. A transformation between the model space and the object space can be determined during the modelling process. This transformation can then be used to transform a model space-based representation of a shape into an object-space representation (and vice versa).

Based on the shape model, different or varying shapes can be generated within the limits of the shape model, e.g. by varying model parameters. Such different shapes can also be referred to as different instances of the model.

In order to establish the shape model, the following steps can be performed. In an alignment step, at least one marking is applied to each of the existing prostheses and a three-dimensional shape of the marked up prostheses is determined, e.g. with a suitable device such as a 3D scanning device which provides scan data representing the shape. Based on the marking, the scan data can be transformed into a reference coordinate system, e.g. a common coordinate system. The marking can comprise a circle at the limbus, i.e. line providing a circular border of the limbus of a prosthesis. Further, the marking can comprise marker or indicators of one or more reference direction(s) such as (a) reference axis/axes, in particular a nasal-temporal (nose to ear) and superior-inferior (up-down) axis. The markings can be applied by a user such as an ocularist.

Using the markings, the determined shape of all prostheses can be aligned in a common reference coordinate system. Alignment can be performed such that for all prostheses, the aforementioned circle at the limbus (which can be identified in the scan data) lies in the same plane and that at least one reference axis, preferably the superior-inferior axis, point in the same direction. It is further possible to mirror the scan data if necessary, so that all sets of scan data represent artificial eyes of the same eye side. For said alignment, it is possible to determine intersection points between the circle and the at least one reference axis, wherein alignment is performed such that intersection points of different scan data sets lie in the same plane. This plane can provide a x-y-plane of a reference coordinate system, wherein the aforementioned reference axis, in particular the superior-inferior axis, can provide a lateral or y-axis, an axis perpendicular to said plane can provide the vertical or z-axis and an axis perpendicular to both of said axes can provide a longitudinal or x-axis of said reference coordinate system. If the y-axis corresponds to the superior-inferior axis, the x-axis can correspond to the nasal-temporal axis. The origin of the reference coordinate system can be provided by the centre of the circle.

It is further possible to perform a correspondence step in order to provide/create corresponding vertices for all scan data sets. In this correspondence step, a depth map or orthographic z-projection of the prosthesis surface in the respective scan data set is determined. In such a 2D representation, the x- and y-coordinate of a pixel in the map can correspond to the x- and y-coordinate in the reference coordinate system, wherein an intensity of the pixel can correspond to the z-coordinate in the reference coordinate system. Said map or projection can be determined for at least one of the anterior surface and the posterior surface, preferably for both.

Starting from the origin, a predetermined number, e.g. 8, of straight radially oriented lines, i.e. directed from the origin to the edge of the map or projection is determined. These lines can be arranged equiangularly along a circumferential direction.

Then, vertices are placed at ratios of a set of predetermined ratios along each of these lines. Values of said ratios can e.g. be chosen in the interval from 0 (inclusive) to 1 (inclusive), wherein a ratio of 0 defines a vertex at the origin and a ratio of 1 defines a vertex at the intersection of the line with the edge of the map. A set of predetermined ratios can comprise at least one value, preferably more than one value. The number of elements in such a set as well as the values of ratios can be different for each line, i.e. line-specific ratios can be assigned to a line. It is, however, possible that the ratios for lines of different subsets of lines are equal, wherein such a subset comprises at least two lines. As a result, different number of vertices can be placed at different angles.

For the map or projection of the anterior surface, the ratios for each line can be chosen such that one vertex is placed on the circle at the limbus. It is possible to divide each line into two segments, wherein the first segment (inner segment) extends from the origin to the circle at the limbus and the remaining segment (outer segment) extends from the circle at the limbus to the intersection of the line with the edge of the map. Then, vertices are placed at ratios of a set of predetermined ratios along each of these segments. The number of elements in such a set as well as the values of ratios can be different for each segment. It is, however, possible that the ratios for segments of different subsets of all inner segments are equal, wherein such a subset comprises at least two inner segments. It is further possible that the ratios for segments of different subsets of all outer segments are equal, wherein such a subset comprises at least two outer segments. As a result, different number of vertices can be placed at different angles on the anterior surface.

As a result, a prosthesis-specific set of vertices is determined for each existing prosthesis wherein said vertices
- represent the shape of the prosthesis (since the vertices represent surface points) in the object space,
- correspond to vertices in a set of a further prosthesis and
- are arranged in a common coordinate system, i.e. the reference coordinate system.

This shape representation can be referred to as uniform shape representation in the context of this invention. In particular, the vertices placed on the circle of the limbus in the projection of the anterior surface can be assigned to the iris region and/or the sclera region and/or the cornea region. It is e.g. possible to assign a vertex to two or more regions simultaneously. Alternatively, a first instance of a vertex can be assigned to a first region, wherein a second (duplicate) instance of the vertex can be assigned to a different region (and a further duplicate instance to a further different region). In the latter case, the different instances of a vertex can be merged at a later point in time, e.g. when fusing the shape information with the appearance information.

Vertices placed outside the circle (in a projection plane which is perpendicular to the vertical axis) can be assigned to the sclera region and vertices inside the circle can be assigned to the cornea region. Such assignment or labelling information can be used for fusing.

It is possible to use the vertices of all prosthesis-specific sets to establish the shape model as outlined above. In other words, the alignment and correspondence step is performed to generate training data for the shape model. In this case, the output of the shape model, i.e. an instance of the shape model in the object space, will provide a shape according to the uniform shape representation.

Determining the (resulting) shape of the ocular prosthesis depending on measured shape information as well as based on shape model can then comprise determining said (resulting) shape as a shape according to the shape model, i.e. the shape of an instance of the shape model, that fits the measured shape information. In particular, the (resulting) shape of the ocular prosthesis can be determined as a shape according to the shape model, i.e. as an instance of the shape model, which minimizes the deviation between the shape, i.e. the model-based shape, and the measured shape information or reduces said deviation to at least a predetermined deviation threshold, in particular in the object space. If the shape model is a parameterized model, this determination can comprise adjusting at least one model parameter such that a cost function is minimized or reduced to at least a predetermined cost function threshold, wherein the cost function value can represent the deviation between the shape model instance (generated according to (a) selected model parameter(s)) and the measured shape information. In other words, an optimization can be performed in which at least one model parameter provides the parameter to be optimized. In the case of the PCA-based shape model, the model parameter can be a weight assigned to a principal component and used in the linear combination of said components providing the model shape. In case of a neural network, the model parameter can e.g. be weights or the aforementioned latent variables. The (resulting) shape can be determined as a vertex-based representation comprising the aforementioned vertices aligned in a reference coordinate system and corresponding to vertices of existing prostheses.

As the scan of the eye socket usually provides only information of a small surface section of the prosthesis, the proposed method allows to determine the overall shape such that a good fit to the scanned eye socket as well as a good fit to the remaining parts of the eye is provided. This results in a shape of the prosthesis which is comfortable to wear for the patient.

The proposed method advantageously allows to automatically generate the shape of a prosthesis based on incomplete surface data which is extracted from volumetric scan data of the eye socket or eye surface in an accurate manner which provides a good wearing feeling for the patient.

The determination of the shape of the ocular prosthesis according to a second aspect of the invention comprises generating shape information of an existing patient-specific prosthesis and transforming it into a uniform shape representation. The existing patient-specific prosthesis can be a prosthesis which is to be replaced. It can also be a prosthesis whose shape was determined according to the first aspect (see above) but where the shape had to be (heavily) modified by the ocularist during the fitting appointment. Depending on the modifications, or adjustments, the ocularist does, the appearance of the prosthesis can be corrupted, either because he cuts in the color layer or because material such as wax is added to make the shape bigger. This advantageously allows to feed back modifications that an ocularist does for a patient's prosthesis such that a new prosthesis with a desired appearance and a shape that fits can be produced.

Corresponding to the proceedings with respect to the alignment step outlined before, at least one marking can be applied to the existing prosthesis and a three-dimensional shape of the marked up prosthesis can determined, e.g. with a suitable device such as a 3D scanning device which provides scan data representing the shape of the existing prosthesis. Then, said scan data can be transformed into the common reference coordinate system as outlined before. Corresponding to the proceedings with respect to the correspondence step outlined before, a set of vertices can be determined. Then, the (resulting) shape of the ocular prosthesis is determined as the uniform representation of the patient-specific prosthesis, in particular as a vertex-based representation comprising the aforementioned vertices aligned in a reference coordinate system and determined in order to correspond to vertices of further prostheses.

In other words, the scan of a prosthesis shape is aligned and processed in this way and then is used as the (resulting) shape of the prosthesis.

The determination of the shape in the uniform shape representation has the advantage that either the shape of the ocular prosthesis determined according to the first aspect or the shape of the ocular prosthesis determined according to the second aspect can be used to be fused with the appearance information which increases the applicability of the proposed method. This e.g. allows to feed back modifications of an ocularist to an existing prosthesis as explained above. Further, the uniform representation allows to easily identify correspondences between regions in the determined shape and the determined appearance. This is in particular the case because vertices in the shape according to the uniform representation are placed at the circle at the limbus which allows an easy identification of the sclera, the iris and a cornea region in the determined shape. Further, an easy modification of the sclera, the iris and the cornea region is allowed. Such a modification can e.g. be a normalization of the cornea and the limbus by which the vertices can be adjusted in order to have the limbus lying within a plane spanned by the aforementioned lateral and longitudinal axes and/or in order to have the cornea describe a desired, predetermined dome shape. Adjustment can also be performed to modify or scale the limbus and cornea region such that the iris fits in the limbus circle.

The benefit of using a uniform shape representation is such a post-processing step can be applied regardless of the shape data source, be it a a shape determined according to the first aspect or the shape of the ocular prosthesis determined according to the second aspect.

The transformation into an uniform representation also allows to efficiently correct small errors in the scan data, such as erroneous markings of the limbus resulting in a too small or too big iris, or correction of scan artifacts that can occur when scanning the transparent cornea dome.

Another advantage of transforming transforming into a uniform shape representation is an increased reliability of determining the iris plane. Some 3D-scanning devices can have troubles determining the surface of transparent objects, which in the case of eye prostheses can be the cornea. In that case the surface has to be covered with e.g. an opaque spray, which then allows to capture the shape of the cornea but blocks the iris from being captured. Without the iris being visible it becomes difficult to correctly determine the iris plane and any errors here can affect the angle of gaze (i.e. the prosthesis may appear to look in a different direction than the companion eye). By the uniform shape representation, the information on the iris plane is readily provided and the above mentioned affection of the angle of gaze is avoided.

If the shape of the ocular prosthesis is determined according to the second aspect, i.e. by scanning an existing patient-specific prosthesis and transforming it into a uniform shape representation, it is also possible to feed such information back into the generation of the shape model. It is e.g. possible to check if the (scanned) shape of the ocular prosthesis deviates from the shape model more than a predetermined extent. If this is the case, the uniform representation can be added to the training data. A deviation can e.g. be determined in the model space or in the object space. If e.g. a transformation from the object space into the model space is known, e.g. the PCA-based transformation, such a transformation can be applied to the uniform representation of the scanned existing prosthesis and a deviation can e.g. be detected if at least one of the weights of the transformed representation is outside a predetermined interval of weight values.

Also, subsequent to the transformation into the model space, the transformed representation can be back transformed into the object space and a deviation can e.g. be detected if the resulting shapes, i.e. the shape of the existing ocular prosthesis and the back transformed shape, differ from each other more than a predetermined amount.

It is further possible that at least one quality criterion is evaluated for a determined (resulting) shape of the ocular prosthesis, wherein the determined shape is discarded in case the at least one quality criterion is not fulfilled and a further shape determination is performed. Such a further shape determination can e.g. involve the variation of at least one optimization parameter different from the optimization parameters in the previous determination. In particular, such a further shape determination can involve varying at least one alignment parameter which will be explained in the following. It can also involve using a different reference shape which will also be explained in the following.

In addition or as an alternative to the outlined determination of the shape of the ocular prosthesis, the generation of appearance information comprises performing at least one of
- a color characterization being performed using the same or similar viewing conditions as a color characterization of a device used for the manufacturing of the ocular prosthesis based on the appearance information and
- an inhomogeneous illumination correction.

In general, the generation of appearance information comprises color imaging the patient's eye which can be the eye to be replaced or the companion's eye. Such a two-dimensional image can be generated by suitable means, in particular an imaging device which can also be referred to as appearance imaging device. Such an imaging device can be a camera, e.g. a CCD- or CMOS-based RGB camera. It is, however, also possible to use a spectral camera as imaging device, wherein the spectral camera can also be a CCD- or CMOS-device capturing more channels. Imaging can be performed under predetermined physical imaging conditions, e.g. with predetermined imaging parameters such as a predetermined focus value, aperture value, working distance, exposure time etc. and lighting conditions, e.g. a predetermined intensity and (a) predetermined spectral power distribution.

The shape scanning device and the appearance imaging device can be separate devices. Both devices, however, can be integrated into one imaging unit, e.g. into a common housing of one imaging unit, wherein said imaging unit can provide both, the shape and the appearance information. In such an imaging unit, both device can be co-registered which can mean that a spatial relation between said devices, in particular of device-specific coordinate systems, is known. In this case, it is also possible that the aforementioned mesh information a co-registered with the image provided the appearance imaging unit.

Color characterization for the imaging device for capturing the image of the patient's eye can be performed before capturing the image of the patient's eye. This can involve capturing images of targets with known, predetermined colors, wherein these images are captured preferably under the same physical imaging conditions, in particular lighting conditions.

These physical imaging conditions preferably match the viewing conditions, i.e. are similar to said conditions, employed by or for the color characterization of a device used for the manufacturing of the ocular prosthesis. In other words, the physical imaging conditions or a subset of said conditions, in particular the spectral power distribution of the illumination, the observing characteristics and imaging device spectral sensitivities, are adapted to the viewing conditions under which the color characterization of the manufacturing is performed. If physical imaging conditions for the color characterization of the appearance imaging device and the viewing condition of the manufacturing device are exactly the same, a 1 to 1 mapping from RGB camera colors to CIELAB color for the printing/manufacturing conditions is possible. Differences in the physical imaging conditions can generate a metamer mismatch. In this case, two different physical materials can create the same RGB value when captured under the physical imaging conditions but have different CIELAB values under the printing/manufacturing conditions.

In the context of this invention, similar physical imaging conditions can denote physical imaging conditions which are identical or which do not deviate more than a predetermined amount from each other, e.g. not more than an amount which would result in a different color characterization.

The color chart that defines the set of targets should ideally include the colors that cover the population's iris and sclera colors. In other words, the colors used for the color characterization are selected in a population-specific manner, in particular with the population to which the patient belongs to. Preferably, some or all of the colors for the characterization are selected from a set of iris or sclera colors that appear most frequently in a particular population group.

Capturing the eye image and the color characterization images is also preferably performed under the same physical imaging conditions, in particular the same lighting conditions. Preferably, capturing the eye image and the color characterization images is performed in a dark room with only the light source of the imaging or scanning device, e.g. the OCT device, being activated in order to avoid uncontrolled stray light. Also, the distance of the iris to the camera and to the light sources should match the distance of the targets to the camera and the light sources during the characterization process.

The color characterization can then be determined by identifying a transformation or a sequence of transformations from the image-device-dependent color information such as RGB values to device-independent color information such as CIEXYZ or CIELAB values.lt is e.g. possible to identify or compute transformations of a two-stage color characterization, where the first transformations converts an image-device-dependent color information such as RGB values to an image device-independent color information such as CIEXYZ values. A second transformation can provide a correction of image device-independent color information in the device-independent color space such as the CIELAB color space. Various types of transformations can be applied, e.g. linear, polynomial or rootpolynomial transformations. The explained two-stage color characterization is, however, only an example. These transformations can be fitted to pairs of image-device-dependent color information and corresponding device-independent color information. The image-device-dependent color information can be extracted from patches of the captured color chart and can e.g. be RGB values. Image device-independent color information can be obtained by color measurements of the same chart patches, e.g. by a spectral photometer, under the assumption of predetermined viewing conditions for graphic technology and photography which are e.g. defined in applicable standards such as ISO 3664:2009 "Graphic technology and photography - viewing conditions". Viewing conditions can comprise condition on the illumination, e.g. a D50 illumination, and the observer, e.g. a 2° observer.- Viewing conditions can e.g. comprise color matching functions related to the spectral sensitivity of a predetermined observer as well as illuminant information.

It is, of course, possible to use different, alternative color characterization methods known to the skilled person.

The appearance information is then extracted from such a color characterized eye image, in particular as the device-independent color information resulting from applying the color characterization transformation on the device-dependent color information.

According to a first aspect of the generation of appearance information, the color characterization is performed using the same or similar viewing conditions as for a color characterization of a device used for the manufacturing of the ocular prosthesis based on the appearance information. These viewing conditions can be selected or predetermined, in particular by a user. Using the same or similar viewing conditions can mean that the same or similar imaging viewing conditions are considered computationally, e.g. in the transformation into a device-independent color space, while the physical imaging conditions may be different or while the same or similar physical imaging conditions are actually set for performing the color characterization. As viewing conditions or in the context of the viewing conditions, it is e.g. possible to consider a D50 illumination and a 2 degrees observer according to the CIE standard. As outlined above, viewing conditions are considered to compute the transformation of the device-dependent color information to the device-independent color information, for the characterization of the appearance imaging unit as well as to compute the transformation of the device-independent color information to the device-dependent color information to characterize the manufacturing system. This advantageously allows to avoid the fact that the output of the color characterization transformation under the assumed condition A is misinterpreted by the printing/manufacturing system to be for condition B. The manufactured color of the eye prosthesis can then mismatch with the companion eye under conditions A and B.

Similar viewing conditions can be provided if a similarity measure is higher than a predetermined threshold value, wherein increasing values of said measure represent an increasing similarity. Such a similarity measure relating to a similarity of different lighting conditions can e.g. be the color rendering index (CRI) and an exemplary value can be 0,95. Another similarity measure can be the so-called Vora value which is defined in the document P. L. Vora and H. Joel Trussell, "Measure of goodness of a set of color scanning filters," JOSA A, 10, No. 7, 1499-1508 (1993). The Vora value represents the similarity between lighting matrices, wherein a lighting matrix is the product of the observer's color matching functions (CMFs) and the spectral power distribution of the illumination (illuminant). An exemplary threshold value related to the Vora value can be 0.9. This allows to transform the image into a device independent color space defined for a similar observer and illuminant as used for the color characterization of a device used for the manufacturing of the ocular prosthesis based on the appearance information. A color characterization of such a device can be performed e.g. according to requirements set out in the ICC standard.

Performing the aforementioned color characterization allows to improve the appearance of the resulting prosthesis which is produced by the device used for manufacturing.

Color characterization, in particular the determination of the color characterization transformations, are not necessarily required if a spectral camera is used. In this case, capturing a spectrally-known reference white patch can be performed to compute the spectral reflectance as known by the skilled person. From said spectral reflectance, device-independent color information such as CIELAB information can be determined for a given observer, e.g. a 2 degrees observer, and illuminant, e.g. a D50 illuminant.

According to a second aspect of the generation of appearance information, an inhomogeneous illumination correction of the captured image is performed. This can be performed in the image before or after the transformation into the device independent color space is performed. The inhomogeneous illumination correction can also be referred to as flatfielding. Such a correction can be performed by generating an image of a white or gray target, preferably under the same physical imaging conditions, in particular lighting condition, as used for the generation of the eye image. The target can comprise or be provided by a flat surface. It is, however, also possible that a so-called geometry aware flatfielding is performed. In this case, the target can have a predetermined shape, in particular a curved shape or a shape with curved sections. More particular, the target can have the shape of a conformer or a shape provided according to the shape model. Then, at least on correction value for the generated image can be determined which can be used to correct the generated raw image into a uniform image. It is possible to determine at least one correction value per pixel, preferably two values per pixel, the pixel's gain and its dark current. These values can then be used in a way known to the skilled person in order to determine a corrected image from a raw image generated by the imaging sensor of the imaging device.

Performing the inhomogeneous illumination correction advantageously allows to improve the accuracy of appearance information determination and thus also the appearance of the resulting prosthesis. This is in particular the case if color information of the image are used to render or artificially generate color information of a specific region, for example the sclera region since in the case of a non-corrected image such artificially generated color information will not provide the desired color reproduction of said region. Further, the correction also allows a better segmentation of different regions (which will be explained later).

The proposed method and system allow to compute a 3D model comprising shape and appearance to manufacture an ocular prosthesis for a patient. In other words, the proposed method and system allow to compute a (joint) shape and appearance template to manufacture an ocular prosthesis for a patient.

In a further embodiment, the shape of the ocular prosthesis is further determined based on at least one reference shape. Such a reference shape can provide the basis shape for a conformer shape. Alternatively, the reference shape can be a shape assigned to a conformer shape (and does not necessarily correspond to the aforementioned basis shape but can be a shape similar to the basis shape). The assignment can be a predetermined assignment. In both cases, the reference shape can be referred to as conformer-specific shape.

A conformer can be manufactured by selecting a specific representation of the shape in the shape model space, e.g. by selecting specific model parameters, and then transform this representation into the object space to provide an instance of the shape model. The shape of said instance corresponds to the basis shape of a conformer. Then, this instance can manufactured, e.g. printed. Further, the manufactured instance can be modified, e.g. in order to provide at least one optically detectable landmark (which will be explained later). It is e.g. possible to cut away a part of the front surface and carve out a part of the back surface of said shape. Alternatively, the instance can be modified before manufacturing, e.g. computationally. The shape of the manufactured and, if applicable, modified instance corresponds to the conformer shape. Alternatively, the reference shape can be the conformer shape, i.e. the shape of the manufactured and, if applicable, modified instance of the shape model. As outlined above, such a conformer can provide a temporary prosthesis inserted into the eye for generating the measured shape information. i.e. for scanning.

The reference shape can be determined as the conformer-specific shape of a selected conformer or as a function thereof. It is e.g. possible that the patient and/or the ocularist select one or more conformers which are then used to determine the reference shape.

If the reference shape is determined as a function of the shape of at least one conformer, it is possible to determine the reference shape as an interpolated shape of two or more conformer-specific shapes or as an interpolated shape of a conformer-specific shape and a mean shape of the conformer (which can e.g. correspond to the PCA-based mean shape).

Conformer-specific shape information can be assigned to each conformer and can be stored in a database and may be accessed during the runtime, e.g. using a unique identity of a conformer. Said identity can e.g. be provided by an identifier applied to the conformer, e.g. in form of a serial number, a RFID tag, a barcode or the like. It is, however, also possible to identify the conformer based on an image evaluation wherein the image to be evaluated may be the generated during the aforementioned scanning of an eye socket with the conformer inserted into the eye. It is, however, also possible to generate a further image of the conformer and to perform image-based identification based on an image not generated during the aforementioned scanning of an eye socket with the conformer inserted into the eye. The conformer identity can also be provided by a user input, e.g. via a human machine interface, or by an external system. It is further possible that the conformer identity can be determined based on at least one of the aforementioned markings. Said markings can be extracted and analysed to determine the identity. In this case, a conformer can have unique markings providing a unique identity.

In particular, the conformer can be based on one instance of the shape model which means that the conformer is manufactured/provided according to the shape model. If the model is a parametrized model, the conformer can be based on an instance according to selected or predetermined model parameters. In other words, the conformer shape is derived from the shape model and thus the shape model is used to produce the conformer.

The conformer can be selected such that a patient feels comfortable wearing the conformer. Determining the (resulting) shape of the ocular prosthesis depending on measured shape information as well as based on shape model and the reference shape can then comprise determining said (resulting) shape as a shape according to the shape model that fits the measured shape information as well as the reference shape. In particular, the (resulting) shape of the ocular prosthesis can be determined as a shape according to the shape model which minimizes a fused deviation which represents both, the deviation between the shape, i.e. the model-based shape, and the measured shape information (first deviation) as well as the deviation between the shape, i.e. the model-based shape, and the reference shape, (second deviation) or reduces said fused deviation to at least a predetermined fused deviation threshold. The second deviation can be determined in the model space or in the object space. In order to determine the fused deviation, the first and the second deviation can be weighted, e.g. with predetermined weights, or fused in alternative ways in order to provide a trade-off between both deviations. In this manner, the conformer shape is used in the fitting process.

In particular in the case that the conformer is selected as a conformer which is comfortable to wear for the patient, the proposed method allows to determine the overall shape of the prosthesis such that a good fit to the scanned eye socket as well as a good and comfortable patient-specific fit to the remaining parts of the eye is provided.

In a further embodiment, the reference shape is selected based on at least one existing conformer which is selected from a set of multiple conformers. The set of multiple conformers can comprises a number of m conformers, wherein m can be equal to or larger than 2, 5, 10, 20, 50 or 100. The shape of all conformers of the set can be different but derived from the shape model, i.e. can be different instances of the shape model (which can be additionally modified). Thus, the shape model can be used to produce all conformers of the set. Conformer-specific shape information can be assigned to all conformers and can also be stored in a database and may be accessed during the runtime, e.g. using a unique identity of the conformer which has been explained above.

In this case, a patient and/or an ocularist can select the conformer from the set of all conformers which provides the best wearing feeling and/or provides the best filling of the socket without causing discomfort. Then, this conformer can be identified and the determination of the (resulting) shape of the ocular prosthesis depending on measured shape information as well as based on shape model and the reference shape can be performed as outlined above. This advantageously allows to determine shape information provide a patient-specific fit to the scanned eye socket as well as a good and comfortable patient-specific fit to the remaining parts of the eye.

In a further embodiment, the socket surface shape information and the shape model are aligned in a common reference coordinate system, wherein alignment parameters are determined based on at least one optically detectable landmark of the conformer, wherein the optically detectable landmark is detected in an image providing/encoding the socket surface shape information, e.g. an image depicting the socket surface. The alignment can be performed in the object space.

As mentioned above, the socket surface shape information can be extracted from image data of the eye socket, in particular of an anterior surface an orbital implant, in particular if covered by the conjunctiva, wherein said image data is generated by a scanning device. The image can be generated while the conformer is inserted into the eye socket. The conformer can have or provide at least one optically detectable landmark. Such a landmark can e.g. be a region or section of the conformer with a predefined shape, preferably a flat surface but other embodiments of an optically detectable landmark such as a curved surface, a waved surface, a stepped surface, a surface with elevations and/or deepenings of a predetermined shape, e.g. cuboid, cubic, a pyramid shape, a cylinder shape or the like, a perforated surface are imaginable. The optically detectable landmark is not necessarily a surface and can also be provided by an element or a structure attached to, embedded in or provided by the conformer which can be identified by evaluating image information such as color information, shape information or the like. It is e.g. possible to provide a landmark as a region with predefined optical characteristics (such as scattering, reflecting or color characteristics), e.g. a coloured region, on or within the conformer, wherein said region can also have a predetermined shape which can be used for identification and wherein the predefined optical characteristics are different from the remaining material of the conformer. An exemplary region could be a ring or a torus with predefined dimensions optical characteristics embedded in the conformer material.

By calibrating the scanning device, it is also possible to determine a spatial relationship between the scanned structures, e.g. the measured surface, and the at least one optically detectable landmark. If both, the landmark and a scanned structure are depicted in the image or volume provided by the scanning device, it is possible to determine the relative position and/or orientation of the landmark and the scanned structure, .e.g. a distance along a selected axis. It is also possible to determine a position and/or orientation of the conformer with respect to the reference coordinate system based on the landmark identification.

Preferably, the conformer can provide a window region having a front or anterior surface and rear or posterior surface with predetermined surface characteristics. Preferably, the surfaces can be flat surfaces. The material of the conformer between the two surfaces can have a predetermined or known thickness. The thickness information can be used to separate the surfaces in an image and/or to correct changes of an optical path of the rays used for scanning. The window region can be arranged such that scanning/imaging of the eye socket is performed during this region which e.g. means that radiation used for image generation radiates through the window region. From this it is clear that the material of the conformer, in particular of the window region, is transparent for the radiation used for image generation.

The optically detectable landmark can be designed and/or arranged with a predetermined spatial relation (position and/or orientation) relative to the common reference coordinate system which can be a coordinate system fixed in position relative to the conformer. In this case, one, multiple but not all or all alignment parameters required to define the spatial position and/or orientation of the optically detectable landmark in the common coordinate system can be known. Unknown or undeterminable alignment parameters required to perform the mapping from the optically detectable landmark (and thus also from the measured surface information) can be selected as predetermined standard parameters. It is also possible to use one or more alignment parameter(s) as optimization parameter(s) in the aforementioned optimization, e.g. to minimize a cost function or a difference metric, in particular unknown parameters. Alignment parameters can represent a rotation, particularly around the aforementioned lateral axes, and a translation, in particular along the aforementioned axes.

If the optically detectable landmark is a flat surface, the surface can be orthogonal to the aforementioned vertical axis of the reference coordinate system while the remaining axes are oriented in parallel to the surface. It is further possible that the front and/or the rear surface is arranged at a predetermined distance from the origin of the reference coordinate system, i.e. from the limbus plane, along the vertical axis which advantageously allows to provide a penetration depth sufficient to reliably depict both, the surface(s) of the landmark as well as the socket surface in the scan data. As a result, a depth information related to the vertical axis can be assigned to the socket surface shape information in the reference coordinate system. In other words, an alignment parameter relating to the vertical axis can be determined based on the predetermined distance, e.g. as the predetermined distance.

Alignment parameters with respect to the remaining axes can also be predetermined parameters, in particular as it can be assumed that a patient with an inserted conformer is oriented in a predetermined relative position and/or orientation with respect to the shape scanning device.

It is also possible that conformer markings can be extracted and analysed in the image. In this case, the markings can also provide optically detectable landmarks. The detection of such markings, e.g. by using image processing methods known to the skilled person, can be used in order to identify the position and/or orientation of the socket surface shape information with respect to the reference coordinate system.

This advantageously allows a simple and reliable way of matching the (surface of the) shape model and the measured surface information in a common coordinate system in order to determine the (resulting) shape of the prosthesis.

In a further embodiment, the shape of the (resulting) ocular prosthesis is determined such that a difference metric is minimized, wherein the difference metric is determined as a function of a deviation between the shape of the ocular prosthesis and the socket surface shape. This has already been explained above. The shape of the (resulting) ocular prosthesis is in particular determined according to the shape model. In this case, the deviation can only be determined for a surface region or surface section of the shape model which corresponds to the region/section for which the socket surface shape is determined/measured, e.g. by scanning. Such a region/section can be identified by mapping or transforming the socket surface information and the shape model into a common reference coordinate system as outlined above. The difference metric can correspond to or represent the cost function value of an optimization problem as outlined above. In other words, the shape is determined according to the shape model in such a way that the deviation between the tissue surface, i.e. the anterior surface of an orbital implant or tissue covering the orbital implant, and the rear surface of the prosthesis (or a section thereof) is minimized. The deviation can e.g. be a deviation along a specific axis in the reference coordinate system in particular the vertical axis. If the rear surface of the model and the tissue surface are both provided by a depth map with the depth being measured along the vertical axis and with respect to the x-y-plane of the reference coordinate system, the deviation can e.g. correspond to the sum of the absolute or squared values of all depth value differences or any other energy function representing the deviation. The energy (or cost function) which is to be minimized can therefore contain a term that penalizes shapes of the model that are far away from the scanned socket surface shape.

This advantageously allows a simple and reliable way of considering information provided by the shape model as well as measured surface information in order to provide an ocular prosthesis that has a good overall fit to the scanned eye socket as well as a good fit to the remaining parts of the eye.

In a further embodiment, the difference metric is further determined depending on a second deviation between the shape of the ocular prosthesis and a reference shape provided by/according to the shape model. This and corresponding advantages have already been explained above. The reference shape can be the basis shape of the conformer or a shape assigned to the conformer which is selected by the user as the conformer with the best wearing feeling (see above). In other words, the energy (or cost function) which is to be minimized also contains a term that penalizes shapes that are in the shape model space far away from the reference shape.

It is further possible that the first and the second (and, if applicable, any further) deviation are weighted differently in order to determine the difference metric. Preferably, the first deviation is weighted higher that then second deviation.

It is possible to additionally or alternatively use further penalizing terms when evaluating the difference metric. If alignment parameters are varied as optimization parameters, it is possible to use a term which penalizes an alignment parameter outside a predetermined interval of admissible values. If a scaling factor for the shape is varied as an optimization parameter, it is possible to use a term which penalizes a scaling factor outside a predetermined interval of admissible values.

It is also possible to select multiple reference shapes of the ocular prosthesis, e.g. reference shapes provided by the model which is parametrized with different sets of parameters. Then, for each of said reference shapes, a reference shape-specific shape of the ocular prosthesis can be determined by minimizing the said difference metric. Then, the (resulting) shape can be determined based on the reference-specific shapes, e.g. by fusing the different reference-specific shapes. Such a fusing can be performed by determining the (resulting) shape as an average shape of all the reference-specific shapes or as a weighted average shape, wherein it is possible that predetermined weights are assigned to each reference-specific shape. Alternatively, the (resulting) shape can be selected from the set of reference-specific shapes, e.g. as the reference-specific shape which provides the lowest final metric value. Also, each reference-specific shape can be manufactured and the ocularist and/or the patient can then select one of said shapes, e.g. as the shape that fits best.

In a further embodiment, minimizing the difference metric is performed by varying at least one model parameter, wherein the (resulting) shape of the ocular prosthesis is determined as the shape provided by the shape model using the at least one parameter which minimizes the difference metric, i.e. as an instance of the shape model generated with the at least one parameter. In this case, the model is a parametrized model, e.g. the PCA-based shape model as explained before. It is, however, possible that other parameterized models are used to provide the shape model. In other words, the parameter(s) of the parametrized model provide(s) (an) optimization parameter(s) for the aforementioned optimization problem. In order to determine the at least one parameter, any suitable optimization algorithm can be used. Suitable optimization algorithms for one or multiple parameters are known to the skilled person.

This advantageously allows a reliable and accurate determination of the (resulting) shape of an ocular prosthesis. As known optimization algorithms can be used, it is also possible to provide a computationally fast and effective way of the parameter determination.

In a further embodiment, the socket surface shape information and the shape model, in particular surface information of the shape model, are aligned in a common reference coordinate system, wherein minimizing the difference metric is performed by varying at least one alignment parameter. As outlined before, the alignment can be performed by using alignment parameters which e.g. define the mapping/transformation of the measured surface information into the common reference coordinate system. Alternatively, one, selected but not all or all alignment parameters required to perform said mapping is/are used as optimization parameter(s), in particular in addition to model parameters. One exemplary alignment parameter can be a translation of the socket surface shape information along a z-axis of the common reference coordinate system. Other alignment parameters can be provided by a translation along other axes or by a rotation around one of said axes.

This advantageously improves the reliability and accuracy of the determination of the (resulting) shape of an ocular prosthesis. As known optimization algorithms can be used, it is also possible to provide a computationally fast and effective way of the parameter determination.

In a further embodiment, a transparent layer is added to a least a section of the surface of the determined shape of the ocular prosthesis. In other words, a clear coating with a predetermined thickness can be added to the resulting prosthesis shape. The thickness can vary between different sections of the prosthesis.

This advantageously allows e.g. an ocularist to perform manual shape adaption without destroying the appearance. It is e.g. possible to use the vertex-based shape representation to add the transparent layer. This can be done by generating a displacement map or clear coating map that modifies the determined shape, i.e. the vertex-based shape representation, such that the produced shape contains a layer of clear coating that may locally vary and allows the ocularist to remove clear material in some areas leaving the color information intact. This displacement map can be part of the texture. The clear coating map can e.g. only add a thin layer of clear material at the nasal and temporal side of the front but an increasingly thicker layer towards the top and bottom of the prosthesis as the ocularist usually modifies these areas.

Alternatively or in addition, the determined shape of the ocular prosthesis is adapted according to mesh information. It is possible that at least one section of the surface is fitted to a corresponding section provided in the mesh information, in particular in terms of dimension and/or form. If region-labelled mesh data is provided the determined shape, in particular a position of vertices being assigned or enclosing the region, can be adapted such that a deviation between the regions identified in the determined shape and the corresponding region in the mesh data is reduced or minimized. In the case that an iris mesh is provided, the position of the vertices being arranged on the circle of the limbus can be adjusted such that the updated circle provided by the changed positions is adapted in size and/or form the iris mesh. A similar approach can be taken for if a sclera mesh and/or a cornea mesh is provided. It is possible to only adjust the vertices being arranged on the circle of the limbus. It is, however, also possible to adjust non-limbus vertices, e.g. such that no vertices of the sclera region are in front or in the iris region.

It is also possible to complement or replace vertices of the determined shape by mesh-based vertices, wherein mesh-based vertices can denote vertices which are provided by the mesh information, e.g. vertices on the surface of region-labelled mesh data such as an iris mesh. In case that an iris mesh is provided, the vertices assigned to the cornea in the determined shape can be removed and replaced by vertices on the surface of the iris mesh. Such a shape can provide a first shape object which can be used for fusing with the appearance information. The removed cornea vertices can provide a further shape object, in particular a shape object with transparent characteristics, which can be added to the first shape object. A similar approach can be taken for if a sclera mesh and/or a cornea mesh is provided. Alternatively, vertices on the surface of the iris mesh can be used as an integrated object which complements an object provided by a shape object with the vertices on the sclera, the limbus and the cornea.

It is also possible to add additional vertices and faces when combining different regions, e.g. the iris and sclera region, to close the gap between these.

In a further embodiment, the generation of appearance information further involves at least one of a thermal noise correction, a specularity removal, a vignetting correction, an inpainting for providing image information for an image area which is identified as an image area not mapping a part of the eye to be reproduced, a contrast enhancement, a filtering of an identified region, a reshaping of an identified region and a recoloring of an identified region. Thermal noise correction can comprise generating an image with a lens fully covered and to then estimate the thermal noise in the resulting image. This estimated noise can then be used for image correction of images. It is possible that at least one correction value for an image correction algorithm is determined based on the estimated thermal noise, wherein the image correction algorithm using said at least one value is applied to an image of the eye. The at least one correction value can be stored in a memory unit and can be accessed at runtime. This advantageously further improves the accuracy of appearance information determination and thus also the appearance of the resulting prosthesis.

The removal of specularities can comprise the identification of pixel with intensities that are higher than a predetermined threshold, e.g. at a maximum value. In other words, oversaturated pixel values can be identified. The identified pixels or pixel regions can then be inpainted with an inpainting technique known to the skilled person, e.g. an inpainting algorithm outlined in the document "Alexandru Telea. An image inpainting technique based on the fast marching method. Journal of graphics tools, 9(1):23-34, 2004".

It is also possible to perform inpainting by using a neural network to predict the color of the pixels, i.e. the texture, of the area to be inpainted. Such a use of a neural network is e.g. described in the document "Guilin Liu, Fitsum A. Reda, Kevin J. Shih, Ting-Chun Wang, Andrew Tao, Bryan Catanzaro, Image Inpainting for Irregular Holes Using Partial Convolutions, Proceedings of the European Conference on Computer Vision (ECCV) 2018". The removal of specularities can additionally or alternatively comprise the identification of image structures or regions having/providing a spatial frequency higher than a predetermined threshold value, wherein such structures or regions can then be inpainted with an inpainting technique known to the skilled person, in particular such that the spatial frequency is reduced. Identification of said image structures can be performed by determining the difference between a lightness channel value of a selected pixel and a median filtered lightness channel value of a selected group of pixels and identifying a high-frequency structure/region if said difference is higher than a predetermined threshold value. This advantageously further improves the accuracy of appearance information determination and thus also the appearance of the resulting prosthesis.

The generation of specularities can be reduced or avoided if a polarization or cross-polarization filter are used, in particular for light received by the camera and/or light emitted by a light source.

Inpainting or digital inpainting can denote a conservation process by which deteriorated or missing regions of a generated image are filled in to provide a complete image. Inpainting processes are known to the skilled person and are not the focus of this invention. They can be applied in order to reconstruct missing, damaged, occluded or superimposed regions in the image of the eye. Different types of inpainting processes can be applied, for instance a structural or geometric inpainting, a textural inpainting or a combination thereof. Inpainting can be performed for image regions in which an aperture, an eyelid or an eyelash is depicted or for image regions which depict a specularity. Before inpainting is performed such an image area or region to be inpainted, i.e. a missing, damaged, occluded or superimposed region, can be identified. Such an area is an image area not mapping a part of the eye to be reproduced in the appearance information to be determined. Such an identification can be performed by using image processing methods such as image segmentation methods known to the skilled person. This advantageously further improves the accuracy of appearance information determination and thus also the appearance of the resulting prosthesis.

Inpainting can make use of color information assigned to pixels surrounding the pixel/region to be inpainted. Alternatively or in addition, inpainting can make use of a priori or predetermined color information which can e.g. be provided by a color model of the eye by which predetermined colors are assigned to selected regions of the eye. The color model can be stored in a memory unit and can be accessed at runtime.

A vignetting correction allows to remove vignetting artifacts from the image of the eye. Vignetting means a darkening of the corners of an image compared to its center. It is a common effect in digital imaging and occurs when the pixels at the edges of the sensor don't receive sufficient light. It is possible that at least one correction value for an image correction algorithm is determined based on a reference image, wherein the image correction algorithm using said at least one value is applied to an image of the eye in order to perform the vignetting correction. The at least one correction value can be stored in a memory unit and can be accessed at runtime. This advantageously further improves the accuracy of appearance information determination and thus also the appearance of the resulting prosthesis.

A contrast enhancement improves the perceptibility of objects in the scene by enhancing the lightness difference between objects (here: eye components such as veins and their backgrounds). Contrast enhancements are typically performed as a contrast stretch followed by a tonal enhancement, although these could both be performed in one step. A contrast stretch improves the lightness differences uniformly across the dynamic range of the image, whereas tonal enhancements improve the brightness differences in the shadow (dark), midtone (grays), or highlight (bright) regions at the expense of the lightness differences in the other regions. Contrast enhancement can be performed based on a histogram, in particular a gray-level histogram. Such a gray-level representation of the image is the lightness channel of the image. It is, however, clear to the skilled person that also alternative approaches to convert the generated color image into a grayscale image can be used. Contrast enhancement can be performed as a lightness contrast enhancement, i.e. an enhancement method which only adjusts lightness channel values of image pixels.

The contrast enhancement selectively applied to regions identified in the image such as an iris region, a sclera region and so on. Exemplary regions and their identification will be explained in the following. If a region-specific contrast enhancement is performed, it is possible to determine a mean value of the pixels intensities of the pixels in the region and to then scale the difference to the mean value of each pixel by a factor higher than one.

Contrast enhancement advantageously further improves the accuracy of appearance information determination and thus also the appearance of the resulting prosthesis. Contrast enhancement also compensates for blurring of the printed eye prosthetic texture caused by light transport within the printing materials. This improves details e.g. of the iris structure.

Filtering of an identified region can be performed in order to remove objects or structures which are identified as objects/structures not being part of the identified regions. It is for instance possible to filter a region such as the sclera region by removing all sub regions associated with veins or eye lashes, i.e. sub regions depicting said veins or eye lashes. This filtering can be performed by removing all pixel values which are not within a predetermined interval of admissible values, in particular region-specific values. As an example, the pixel values which have a red hue or are too dark can be removed. The removed pixel values can then be replaced by values determined by an inpainting such as explained before. Filtering can also be performed in order to blur the transition between two identified regions, e.g. by the application of a Gaussian filter. Filtering can also be performed in order to remove image noise, in particular noise which is different from the aforementioned thermal noise. In this case, non-local means denoising can be applied. Such a denoising is e.g. described in the document Antoni Buades, Bartomeu Coll, and Jean-Michel Morel, Non-Local Means Denoising, Image Processing On Line, 1 (2011), pp. 208-212. https://doi.org/10.5201/ipol.2011.bcm_nlm.

Filtering advantageously further improves the accuracy of appearance information determination and thus also the appearance of the resulting prosthesis.

A reshaping of an identified region can comprise moving the boundary of an identified region, in particular depending on mesh information. As explained before, mesh information provided by the imaging device for imaging the eye can be used to identify predetermined regions of the imaged eye. Then, a boundary of a selected region identified based on mesh information can be compared to the boundary of the corresponding region being identified based on the image, i.e. based on image information. Then, the boundary, in particular a course of the boundary, of the corresponding region can be adjusted based on the boundary identified based on mesh information, e.g. such that a spatial deviation is between said boundaries is smaller than a predetermined threshold value or such that the boundary of the corresponding region corresponds to the boundary identified based on mesh information. Further, pixel values of pixels that are newly assigned to a region based on the reshaping can be determined by an inpainting such as explained before or set to a predetermined, region-specific value. In such a way, an iris geometry can be reshaped.

The recoloring of an identified region can comprise determining new color information of the identified region and replace the existing color information by the newly determined color information. In other words, color information of a specific region can be rendered or artificially generated, in particular based on the existing color information of said region. It is possible to identify a set or distribution of pixel color values in an identified region, preferably after the region has been filtered. Such an identification can be performed by a clustering algorithm such as the k-means clustering. It is then possible to extract a base color value from the result of this identification, e.g. a mean value or the brightest value of the k-means clustering. In other words, the pixel color values resulting from the identification, individually or as a combination, can be used to determine a region-specific base color in order to then render a region-specific base layer. Then, existing color information can be replaced by said base color value. Further, the base color value can be adjusted by generating a noise signal, wherein the base color value is adjusted by the noise signal for selected or all pixels of the identified region. Characteristics of the noise signal can also depend on the results of the aforementioned identification. For recoloring the sclera, it is possible to generate a Perlin noise signal. Recoloring, i.e. procedurally generating a texture of an identified region such as a sclera base texture with staining from color information extracted from the image, advantageously decreases the dependency on the visible area of the identified region, i.e. the sclera, in the image. This allows to adjust the staining based on empirical knowledge. In case of the sclera, for instance, the identified region can become very sparse, in particular if veins are filtered out. Further, recoloring can compensate the problem of inhomogeneous illumination, i.e. a darker sclera color on the temporal side in the image does not result in a darker sclera on the temporal side in the print (but distributes this more evenly on the texture).

In a further embodiment, at least one image region is identified in which a part or region of the eye is mapped. In other words, an image region in which a selected part of the eye is depicted is identified. This allows to provide region-labelled appearance information, e.g. color information. It is in particular possible to assign pixels of the image to identified regions. This assignment or labelling information can then be used for the fusing.

The identification of such a part can involve an image segmentation. Image segmentation can refer to a process of partitioning a digital image into multiple image segments, also known as image regions or image objects (sets of pixels). The goal of segmentation is to simplify and/or change the representation of an image into something that is more meaningful and easier to analyse. Image segmentation is typically used to locate objects and boundaries (lines, curves, etc.) of regions in images. More precisely, image segmentation is the process of assigning a label to every pixel in an image such that pixels with the same label share certain characteristics. Parts or regions to be identified comprise the regions of the image in which the pupil, iris, sclera, skin, eye lids, eye lashes or other regions of interest are depicted. Segmentation methods are known to the skilled person. A preferred image segmentation method for identifying eye regions is a region growing algorithm, preferably a watershed algorithm. Such an algorithm can make use of seed pixels. Such seed pixels can be identified by suitable algorithms. In case of an image of the eye, tracing along the horizontal centreline of the image, or a mean reduction along this axis, allows to identify the pupil that is depicted by the pixels with the minimum intensities along said axis and also the sclera that is depicted by the pixels with the maximum intensities to the right and to the left of the pupil pixels. This allows to identify e.g. a seed pixel of the pupil and the sclera. Edges of the iris can be determined based on a gradient of the image, e.g. using a bit-flip search on the gradient. This allows to identify seed pixels of the iris as a pixel located between these edges. If available, the mesh information provided by the imaging device can be used in order to identify a set of seed points for each region of interest, in particular of the iris and pupil. Such seed points can e.g. be identified from a z-projection of the mesh that is aligned with the color image data. The identified seed pixels that belong to the different regions can then be used to iteratively create and refine a segmentation of the regions of interest, e.g. the pupil, iris, sclera and tissue. Optionally, it is possible to use an aperture mask to identify a camera aperture which may be depicted in the image.

Segmentation of regions, in particular circular or elliptical regions such as the iris, can be refined or performed by applying border or edge detection algorithms. If an edge corresponding to the limbus, i.e. the transition of sclera and cornea which borders the iris, is identified, all pixels outside of said edge can be classified or labelled as sclera pixels. If an edge corresponding to the pupil edge, i.e. the transition of pupil to the iris, is identified, all pixels inside of said edge can be classified as pupil pixels. The pixels in between these edges can be classified as iris pixels.

It is also possible to apply the Daugman algorithm, in particular iteratively at multiple resolutions, to identify a circular edge that best approximates the iris boundary or limbus. Alternatively or in addition, a modified version of the Daugman algorithm can be used that modulates the circle into an ellipsis that maximizes the intensity difference between pixels outside and inside the circle while keeping the area of the circle constant. By this, the fact that the iris is not a perfect circle can be reflected. It is further possible to use a so-called circle hough transform to identify said circular edge.

The identified regions by the segmentation can be used to improve the color match of the inpainted areas. It is e.g. possible to shift a mean color of a specular highlight pixel or area within a region to a mean color of the pixels in the region without the specular highlight pixel or area.

The identified regions by the segmentation can be refined or filtered to remove objects or structures not being part of the identified regions, i.e. mislabelled parts, for example veins. Such a filtering can be performed by using a lightness and hue sensitive filter.

Once, different regions are identified, it is possible to perform a region-specific image processing such as thermal noise correction, specularity removal, vignetting correction, inpainting, contrast enhancement, filtering, reshaping and/or recoloring, wherein processing of different regions is performed differently.

In a further embodiment, a vein generation is performed for introducing vein regions into the image. Although it is also possible to identify vein regions by one of the aforementioned algorithms, veins can also be introduced into the image artificially. In particular, the vein generation can make use of a vein recipe, wherein the vein recipe defines at least one fixed vein characteristic. The vein recipe can be stored in a memory and may be accessed during runtime. A vein characteristic can be at least one of a vein thickness, a vein branching factor, a vein orientation factor and a vein depth. The vein branching factor can represent how often the vein branches along its course. The vein orientation factor can represent how high the variation of the direction of extension of the vein is. The vein depth can represent a depth of the vein with respect to the surface of an eye. Said depth information can be used to determine which vein is depicted in the image in case of a vein crossing. Said information can also be used for 3D printing of the vein. Vein characteristics can be adjusted in an image- or eye-specific manner by defining at least one veining parameter. A veining parameter can be predetermined, provided by user input or by image analysis. It is e.g. possible to analyze the region identified as sclera region in order to extract the parameters which are then used to influence the procedural veining network generation and rendering in particular such that the generated texture resembles the veining visible in the color image in terms of vein thickness and density. The veining parameter can be a scaling parameter of a selected vein characteristic.

The vein generation can be performed by a vein growing algorithm, i.e. in an iterative and/or procedural way. This growing starts at a selected seed point (or pixel) which can be predetermined, selected by a user or by image analysis. Seed points can be selected as anatomically motivated seed points, i.e. as points of the anterior ciliary arteries.

In the following iteration steps, further pixels belonging to the vein are identified depending on the vein recipe and the vein characteristics. E.g. depending on a thickness and the direction of growing, one or more pixels in a local and orientation-specific neighbourhood of the currently considered pixel(s) are classified as pixels of said vein. Further, depending on the vein branching factor, this or one of this pixels can be classified as starting point for a branching vein. The growing can stop after a predetermined number of steps or if the thickness becomes smaller than a predetermined threshold value or if a predetermined image region or border is reached such as the limbus.

Introducing veins can additionally be performed in different levels using different vein recipes and/or different veining parameters for each level. In each level the growing can be performed iteratively as outlined above.

It is for instance possible that a vein growing algorithm is performed on a first level or in a first layer. After growing has been stopped, a further vein growing can be performed on a subsequent level or in a subsequent layer. In a subsequent level/layer, the starting points classified in the previous layer can be used as seed points for the growing. Further, the vein recipe and/or different veining parameters can be adapted such that thinner veins as in the previous layer are generated and/or such that there is a higher variation in the direction of extension. In the last layer of the vein introduction, the classification of branching points can be omitted. Preferably, the introduction of veins can be performed on three layers/levels.

Once the vein pixels belonging to one vein have been identified, the vein can be introduced depending on said pixels. This can involve determining a spline representation, in particular a B-spline representation, using the vein pixels.

In other words, a veining network can be generated artificially and can be added to the image, in particular to the sclera region. This network can consist of multiple layers and can be generated using at least a thickness and a branching ratio parameter to modify the network growth and structure as well as the rendering of the network.

Vein pixel colors used to generate the veins can be extracted e.g. from color calibrated images and stored as vein profiles labeled with vein features such as thickness or depth. Then the generated veins can be rendered with a vein profile or combination of vein profiles that match the veins features.

Further, depth information, e.g. in form of a depth map, can be assigned to the generated veins. Such a depth information allows to create a network in which certain veins are located behind other veins.

Further proposed is a computer program product with a computer program, wherein the computer program comprises software means for the execution of one, multiple or all steps of the method of determining shape and appearance information of an ocular prosthesis for a patient according to one of the embodiments described in this disclosure if the computer program is executed by or in a computer or an automation system, in particular at least one step of determining the shape of the ocular prosthesis depending on said shape information, generating appearance information for the ocular prosthesis and fusing the shape and the appearance information (wherein embodiments of said steps have been explained before). The computer or automation system can comprise or be provided by the aforementioned computing unit, wherein the computing unit can be provided or comprise at least one microcontroller or integrated circuit. The computer program can further comprise software means for the execution of one, multiple or all steps (see above) of the method for generating control signals or the method for producing an object according to one of the embodiments described in this disclosure if the computer program is executed by or in a computer or an automation system.

Further described is a program which, when running on a computer, in particular the proposed computer device or system, or in any other suitable automation system, causes the computer or the (automation) system to perform one or more or all steps (see above) of the method of determining shape and appearance information of an ocular prosthesis according to one of the embodiments described in this disclosure and/or to a program storage medium on which the program is stored (in particular in a non-transitory form) and/or to a computer comprising said program storage medium and/or to a (physical, for example electrical, for example technically generated) signal wave, for example a digital signal wave, carrying information which represents the program, for example the aforementioned program, which for example comprises code means which are adapted to perform any or all of the method steps described herein.

This means that the method or parts of the method in accordance with the invention is for example a computer implemented method. For example, all the steps or merely some of the steps (i.e. less than the total number of steps, see above) of the method in accordance with the invention can be executed by a computer, in particular by the disclosed computer device or system. An embodiment of the computer implemented method is a use of the computer for performing a data processing method. The computer for example comprises at least one microcontroller or processor or integrated circuit and for example at least one memory in order to (technically) process the data, for example electronically and/or optically. The processor being for example made of a substance or composition which is a semiconductor, for example at least partly n- and/or p-doped semiconductor, for example at least one of II-, III-, IV-, V-, VI-semiconductor material, for example (doped) silicon and/or gallium arsenide. The calculating steps described are for example performed by a computer. Determining steps or calculating steps are for example steps of determining data within the framework of the technical method, for example within the framework of a program. A computer is for example any kind of data processing device, for example electronic data processing device. A computer can be a device which is generally thought of as such, for example desktop PCs, notebooks, netbooks, etc., but can also be any programmable apparatus, such as for example a mobile phone or an embedded processor. A computer can for example comprise a system (network) of "sub-computers", wherein each sub-computer represents a computer in its own right.

The computer program product advantageously allows to perform a method of determining shape and appearance information of an ocular prosthesis according to one of the embodiments described in this disclosure for which technical advantages have been outlined before. Also described is a computer program product with a computer program, wherein the computer program comprises software means for the execution of one, multiple or all steps (see above) of the method for manufacturing an ocular prosthesis for a patient according to one of the embodiments described in this disclosure if the computer program is executed by or in a computer or an automation system.

Further proposed is a system for determining shape and appearance information of an ocular prosthesis for a patient, in particular for the manufacturing of the ocular prosthesis, the system comprising at least one means for generating shape information for the ocular prosthesis, at least one means for determining the shape of the ocular prosthesis depending on the shape information, at least one means for generating appearance information for the ocular prosthesis by capturing an image of a patient's eye, at least one means for fusing the shape and the appearance information,
characterized in that
the determination of the shape of the ocular prosthesis comprises
   - determining the shape based on a shape model being determined based on the shapes of existing prostheses or
   - determining the shape by generating shape information of an existing patient-specific prosthesis and transforming it into a uniform shape representation, and/or
   the generation of appearance information comprises performing at least one of
   - an inhomogeneous illumination correction and
   - a color characterization being performed using the same or similar viewing conditions as a color characterization of a device used for the manufacturing of the ocular prosthesis based on the appearance information.

Further proposed is a conformer for the use in a method of determining shape and appearance information of an ocular prosthesis for a patient according to one of embodiments described in this disclosure, the conformer having or providing at least one optically detectable landmark, wherein the optically detectable landmark is detectable in an image providing/encoding the socket surface information, e.g. an image depicting the socket surface. Such a conformer and the corresponding conformer-specific features have already been described before.

Further described is a method for manufacturing an ocular prosthesis for a patient, comprising the steps of:
- determining shape and appearance information of the ocular prosthesis according to one of the embodiments described in this disclosure,
- manufacturing the ocular prosthesis according to the shape and appearance information.

The manufacturing can be performed by 3D printing, wherein the shape and appearance information are fused and then used to generate control data for a 3D printing device and the 3D printing device is operated with the control data.

Further described is a system for manufacturing an ocular prosthesis for a patient, comprising a system according to one of the embodiments described in this disclosure and means for manufacturing the ocular prosthesis according to the shape and appearance information. The means for manufacturing can comprise or be provided by a 3D printing system which comprises a 3D printing device and at least one control unit, wherein the control unit is configured to generate a control signal for the 3D printing device based on the shape and appearance information.

The invention is further described with reference to the attached figures. The figures show:
- Fig. 1: a schematic illustration of an example of a prosthetic eye in an anophthalmic eye socket according to the state of the art,
- Fig. 2: a front view of an ocular prosthesis,
- Fig. 3: a top view of an ocular prosthesis,
- Fig. 4: a schematic flow diagram of determining shape information of an ocular prosthesis,
- Fig. 5A: a front view of an ocular prosthesis with markings,
- Fig. 5B: a front view of an ocular prosthesis with further markings,
- Fig. 6A: a front view of an ocular prosthesis with corresponding vertices,
- Fig. 6B: a rear view of an ocular prosthesis with corresponding vertices,
- Fig. 7A: a perspective front view of a conformer,
- Fig. 7B: a perspective rear view of a conformer,
- Fig. 7C: a perspective front view of an iris mesh,
- Fig. 8: an exemplary 2D scan of the eye socket,
- Fig. 9: a schematic flow diagram of determining shape information of an ocular prosthesis according to an alternative embodiment,
- Fig. 10: a schematic flow diagram of determining appearance information of an ocular prosthesis,
- Fig. 11: a schematic flow diagram of determining appearance information of an ocular prosthesis according to a further embodiment,
- Fig. 12: a schematic flow diagram of determining appearance information of an ocular prosthesis according to a further embodiment,
- Fig. 13: a schematic flow diagram of determining shape and appearance information of an ocular prosthesis,
- Fig. 14: a schematic block diagram of a system for determining shape and appearance information of an ocular prosthesis for a patient,
- Fig. 15: a schematic block diagram of a system for manufacturing an ocular prosthesis for a patient.
- Fig. 16: a schematic flow diagram of a system for manufacturing an ocular prosthesis for a patient.

In the following, the same reference numerals denote the same or similar technical features.

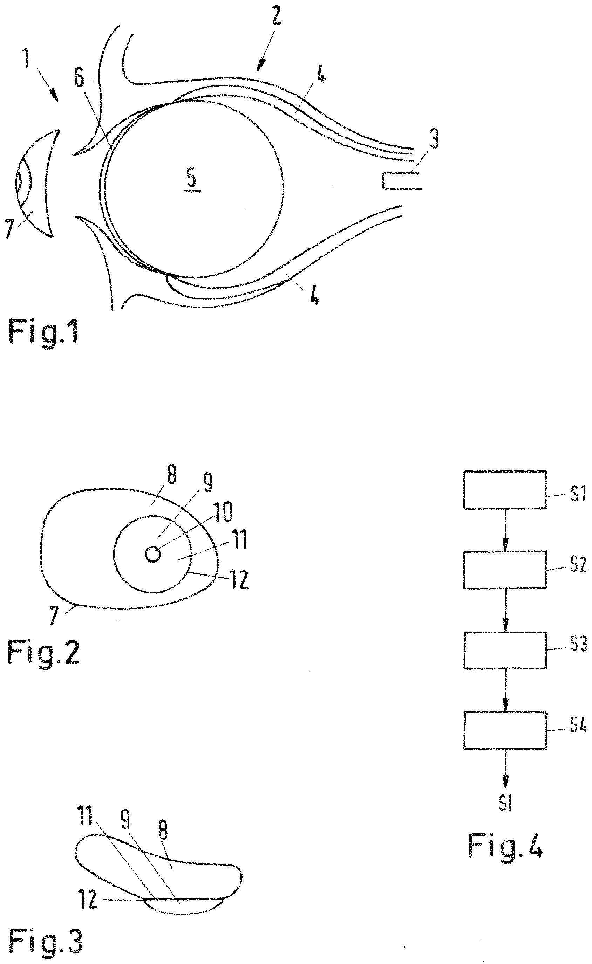

Fig. 2 shows a front view of an ocular prosthesis 7 and Fig. 3 shows a top view of said ocular prosthesis 7 having a portion corresponding to the sclera 8, a portion corresponding to the cornea 9, a portion corresponding to the pupil 10, a portion corresponding to the iris 11 and a portion corresponding to the limbus 12.

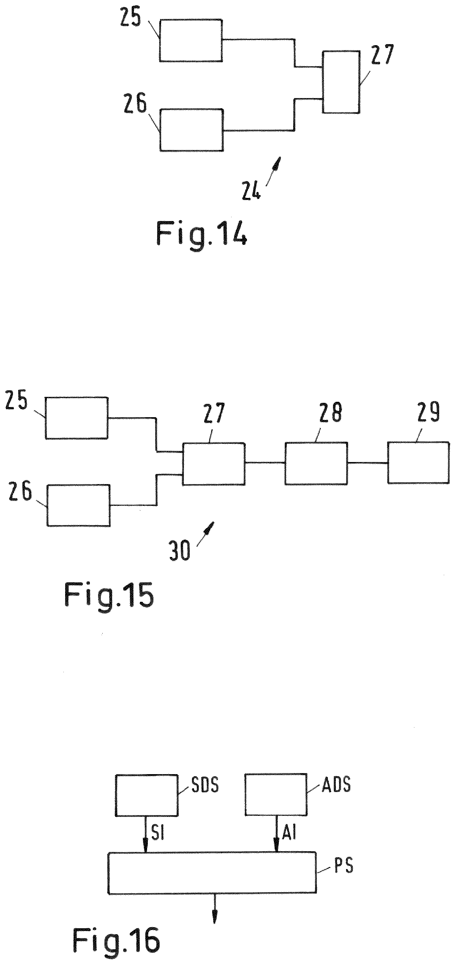

Fig. 4 shows a schematic flow diagram of determining shape information SI of an ocular prosthesis 7 (see Fig. 1). In a first step S1, socket surface information (measured shape information) are generated by scanning the eye socket 2, in particular by using a shape scanning device 25 (see Fig. 14). Thus, volumetric image data of the eye socket 2, in particular of an anterior surface an orbital implant 5, in particular if covered by the conjunctiva 6 (tissue) is generated. Generating the image data (scan data) can be performed while an orbital implant and a temporary prosthesis (which will be also referred to as conformer 19) is in place.

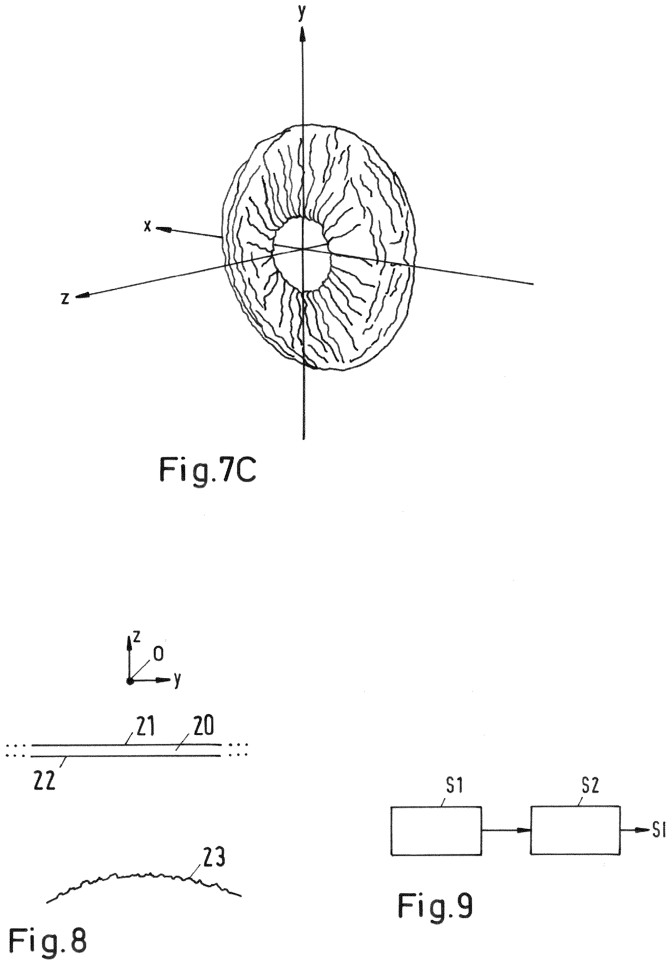

As a result of the first step S1, raw socket volume scan data is provided. In a second step S2, the socket surface on which the prosthesis 7 is to be fitted is identified in the raw data provided by the first step S1. This can also be referred to as socket surface extraction. In a third step S3, the determination of the resulting shape can be performed. Determining the (resulting) shape of the ocular prosthesis 7 can be performed depending on the measured shape information, i.e. the socket surface information, as well as based on a shape model. In particular, said determination can comprise determining said (resulting) shape as a shape instance according to/of the shape model that fits the measured shape information. In particular, the (resulting) shape of the ocular prosthesis 7 can be determined as a shape according to the shape model which minimizes a difference metric, wherein the difference metric is determined as a function of a (first) deviation between the shape of the ocular prosthesis (which is determined according to the shape model) and the measured surface shape information. Optimization parameters can be the model parameters of a parametrized model, wherein said optimization parameters are varied in order to minimize the difference metric. In other words, the fitting procedure finds a shape according to the shape model that best matches the extracted partial socket surface. It minimizes energy that foremost penalizes the difference between the surface of the prosthesis 7 (which is provided according to the shape model) and the surface of the socket 2, the energy can be minimized by variation of the shape e.g. with the L-BFGS algorithm. To determine the deviation, the socket surface 23 (see Fig. 8) can be converted in a 2D depth map and is the compared with the depth map or z-projection of the back of the prosthesis shape. The output of the third step S3 can be information on the resulting shape.

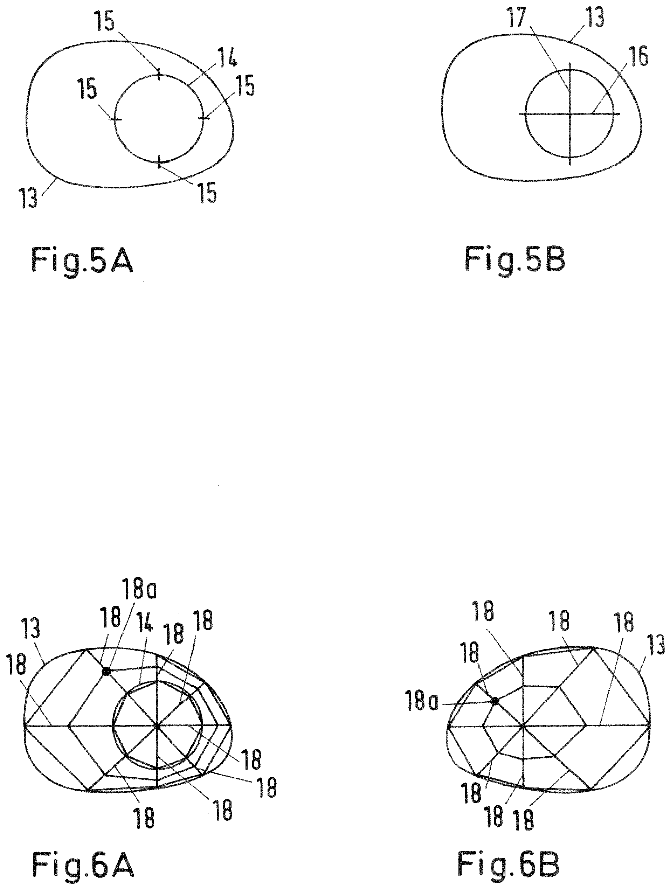

The shape model can be determined based on the shapes of existing prostheses 13 (see Fig. 5A) prior to the determination of the shape information SI shown in Fig. 4. One exemplary way to establish the shape model is to generate shape information, e.g. by 3D scanning, of existing prostheses 13, align said shape information in a common reference coordinate system and determine corresponding vertex points in all these prosthesis-specific data sets. Then, said corresponding vertices of all prosthesis-specific sets to establish the shape model. This shape model can e.g. be a PCA-based shape model which can be established as explained above. Generation of shape information of existing prostheses 13 can involve 3D scanning of these existing prostheses 13. Before scanning, markings can be applied to these prostheses, e.g. by an ocularist. Fig. 5A shows an exemplary marking that is provided by a circle 14 at the limbus, i.e. line providing a circular border of the limbus of an existing prosthesis 13. Further, the marking can comprise marker or indicators 15 of one or more reference direction(s) such as (a) reference axis/axes, in particular a nasal-temporal (nose to ear) and superior-inferior (up-down) axis 16, 17 shown e.g. in Fig. 5B.

Based on the marking, the scan data can be transformed into a reference coordinate system, e.g. a common coordinate system. Alignment can be performed such that for all prostheses 13, the aforementioned circle 14 at the limbus (which can be identified in the scan data) lies in the same plane and that at least one reference axis, preferably the superior-inferior axis 17, point in the same direction. The plane in which the circle 14 is arranged can provide a x-y-plane of a reference coordinate system, wherein the aforementioned reference axis 17 can provide the longitudinal or x-axis, an axis perpendicular to said plane can provide the vertical or z-axis and an axis perpendicular to both of said axes can provide a lateral or y-axis of said reference coordinate system. The origin of the reference coordinate system can be provided by the centre of the circle 14.

For the determination of corresponding vertices, a depth map or orthographic z-projection of the prosthesis surface in the respective scan data set is determined. In such a 2D representation, the x- and y-coordinate of a pixel in the map can correspond to the x- and y-coordinate in the reference coordinate system, wherein an intensity of the pixel can correspond to the z-coordinate in the reference coordinate system. Said map or projection can be determined for at least one of the anterior surface and the posterior surface, preferably for both. Starting from the origin, a predetermined number, e.g. 8, of straight radially oriented lines 18, i.e. directed from the origin to the edge of the map or projection is determined. These lines can be arranged equiangularly along a circumferential direction. Fig. 6A shows a possible distribution of lines 18 for the anterior surface of an existing prosthesis 13, while Fig. 6B shows a possible distribution of lines 18 for the posterior surface of the existing prosthesis 13. The Figs. 6A and 6B also show that vertices are placed at ratios of a set of predetermined ratios along each of these lines, wherein the vertices 18 are connected by line segments. In both figures, one exemplary vertex 18a is referenced by a referenced numeral. Fig. 6B shows that values of said ratios can e.g. be chosen in the interval from 0 (inclusive) to 1 (inclusive), wherein a ratio of 0 defines a vertex at the origin and a ratio of 1 defines a vertex at the intersection of the line with the edge of the map. A set of predetermined ratios can comprise at least one value, preferably more than one value. The number of elements in such a set as well as the values of ratios can be different for each line, i.e. line-specific ratios can be assigned to a line. It is, however, possible that the ratios for lines of different subsets of lines are equal, wherein such a subset comprises at least two lines. As a result, different number of vertices can be placed at different angles.

In Fig. 6A each line is divided into two segments, wherein the first segment (inner segment) extends from the origin to the circle at the limbus and the remaining segment (outer segment) extends from the circle 14 at the limbus to the intersection of the line with the edge of the map. Then, vertices are placed at ratios of a set of predetermined ratios along each of these segments. For the inner segments, the set of ratios comprises the values [0,1]. For the outer segments, the set of ratios comprises the values [0.5,1]. In Fig. 6B, the set of ratios for each line comprises the values [0, 0.5, 1].

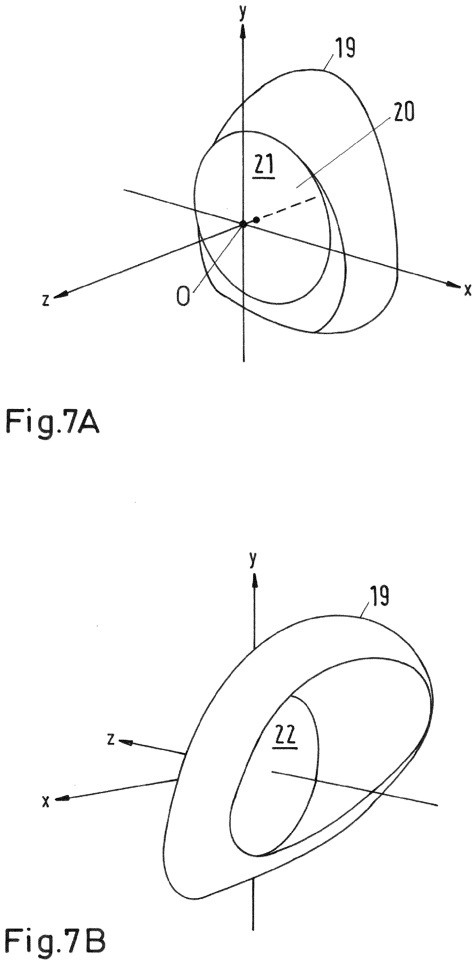

Alternatively, the difference metric used in step S3 is further determined depending on a second deviation between the shape of the ocular prosthesis to be determined and a reference shape. The reference shape can be the shape of a conformer 19 (see Fig. 7A), a basis shape of a conformer 19 which is designed according to the shape model or a shape designed according to the shape model and assigned to the conformer by a predetermined assignment. Said conformer 19 can be selected from a set of existing conformers, in particular as the conformer which provides the best wearing feeling for the patient. In this case, the energy also penalizes shapes that are too far away from the reference shape. This can be done by either penalizing the difference between the surface of the prosthesis 7 (which is provided according to the shape model) and the surface of the reference shape or, in the case of a PCA-based model by penalizing a deviation of the model parameters that are varied in the fitting optimization from the model parameters of the reference shape.

Evaluating the difference metric, in particular the aforementioned deviations, can comprise aligning the socket surface information and the shape model in a common reference coordinate system which has been explained above. Alignment parameters can be determined based on at least one optically detectable landmark of the conformer, wherein the optically detectable landmark is detected in the image providing/encoding the socket surface information.

If the resulting shape determined in the third step S3 is unlikely, e.g. in the case that the determined model parameters are outside a predetermined interval of admissible values, the third step S3 can be repeated using different initial values for the model parameters. It is also possible to introduce further optimizing parameters as alignment parameters which represent a rotation, particularly around the aforementioned lateral axis, and a translation, in particular particularly along the vertical axis. In this case, the energy, i.e. the difference metric, also penalizes translations and rotations away from the initial orientation and position.

In a fourth step S4 which is optional, shape post processing can be performed in order to provide the (resulting) shape information SI which are then used for manufacturing the prosthesis 7. It is e.g. possible to modify the determined shape such that the produced shape contains a layer of clear coating that may locally vary and allows the ocularist to remove clear material in some areas leaving the color information intact.

Once a shape is found a set of post-processing steps are preformed, the cornea dome is fitted to the mean shape dome or the patient's cornea information, this also includes fitting the limbus to the iris size. This has been explained before.

The resulting shape can be smoothed with a subdivision scheme and split along the limbus to separate the cornea and (effectively sclera) eyeball shape. The sclera shape can then be UV-mapped with a cylindrical or spherical projection.

Fig. 7A shows a perspective front view on the anterior surface of a conformer 19. This conformer 19 can be inserted into the eye while generating the scan data in step S1. Further shown are the axes of the reference coordinate system. The conformer has or provides a conformer window 20 wherein said window 20 is a section with a flat front (anterior) surface 21 and a parallel rear (posterior) surface 22 (see Fig. 8). The remaining shape of the conformer 19 is adapted such as to fit into the eye socket 2. The shape of the conformer 19 can e.g. be determined based on an instance of the shape model, in particular with a selected set of model parameters. Then, as a modification of said shape model instance, a conformer window 20 can be introduced and the conformer 19 can be manufactured accordingly. The conformer window 20 provides an optically detectable landmark of the conformer 19. The distance of the conformer window 20 from the limbus plane, i.e. the x-y-plane of the reference coordinate system, along the vertical axis z can be known. Also, the surfaces of the conformer window 20 can be oriented perpendicular with respect to the vertical axis z. In addition to the vertical axis z, Fig. 7A also shows a longitudinal axis x and a lateral axis y which can correspond to a nasal-temporal axis and to a superior-inferior axis respectively. While Fig. 7A shows the front or anterior surface 21 of the conformer 19, Fig. 7B shows a rear or posterior surface 22 of the conformer.

It is further shown that the front surface 21 of the conformer 19 is displaced with a predetermined offset value along the direction of the vertical axis z from an origin O of the reference coordinate system, wherein the front surface 21 is arranged behind the origin O.

The conformer 19 can serve two purposes. First, the socket surface extraction performed in step S2 can involve the identification of conformer parts depicted in the scan data. If e.g. the conformer 19 with a conformer window 20 such as shown in Fig. 7 is used, the conformer window surfaces 21, 22 can be identified in the scan data. Fig. 8 shows an exemplary 2D scan of the scan data which can correspond to a slice of the volumetric scan data. Shown is the conformer window 20 with the front and the rear surface 21, 22 and the vertical as well as the lateral axis z, y. Further shown is the tissue surface 23 which corresponds to the socket surface. Once the surfaces 21, 22 have been identified, it is possible to only consider data information behind said surfaces 21, 22 (with respect to the vertical axis z oriented from posterior to anterior) for identification of the socket surface 23, the remaining scan data can be discarded. Second, the conformer 19 allows to align the socket surface in the reference coordinate system. The extracted window surface can e.g. be used to determine a translation along the vertical axis and thus allows to map the socket surface into the reference coordinate system.

Further, noise in the scan data can be removed, e.g. by noise filtering. Also, (down)sampling of the scan data can be performed. It is further possible to perform identification of the conformer parts in a data set that is sampled with a first sampling rate, wherein identification of the socket surface 23 is performed in a data set that is sampled with a second sampling rate being lower than the first sampling rate. The first sampling rate can be chosen such that conformers parts, e.g. surfaces 21, 22 of the conformer window 20, are preserved in the data (while noise is not or only minimally reduced), while the second sampling rate is chosen to remove more noise and give a better representation of the socket surface data to extract.

The extraction of the conformer window 20 and socket surface 23 can e.g. be done via a column tracing, starting from different pixels at the edge of the volume, that are believed to be outside of the eye socket 2, trace along the depth or z-axis until a surface point is detected. In case the conformer window 20 has a planar surface, iteratively fit a plane until all detected surface points believed to belong to the front or anterior surface 21 of the conformer window 20 lie in a certain distance to the plane. Alternatively other parameter estimation methods, such as RANSAC, could also be used to fit the conformer anterior surface 21 to the detected surface points. Repeat the procedure for the back of the window starting from pixels at an opposite edge of the volume in order to identify the posterior surface 22.

In case of a planar conformer window 20, an additional constraint is that the orientation of the planes for the front and back surface 21, 22 should coincide. This extracted back or posterior surface 22 can also be used to form a mask for tracing the socket surface 23, by only considering columns where the position that was found for the back of the window is within a certain distance to the fitted socket surface 23.

Optionally, an artifact elimination can be performed based on a gradient representation of the volumetric scan data, in particular in the case that such data is AS-OCT data as signals out of the range of the image can show up inverted. In such a case, the signal gradient is also inverted which allows to detect the artifacts by e.g. finding image columns where the signal of the socket becomes stronger with increasing depth.

This extracted surface data can be corrected for some small effects, such as the distance of the conformer window 20 to the limbus plane, changes of speed of light in the conformer window 20, optical refraction of the light direction at the conformer window 20, and the angle of gaze.

Information of the used conformer 19 can be supplied externally, for example providing the ID that then can be used to look up the conformer-specific shape information in a library. Alternatively, using an image of the conformer 19 in the eye socket 2, markings on the window 20 and/or landmarks of the conformer 20 can be extracted. From these markings and/or landmarks the used conformer can be identified. Also, its spatial position and/or orientation can be determined, in particular in the reference coordinate system. With this knowledge a new virtual conformer can be created by reconstructing the used conformer, transforming it with the extracted rotation and translation, and application of the alignment and correspondence procedure for the scans. This gives then a shape representation in the shape model space that serves as a reference shape for the fitting procedure.

Fig. 9 shows a schematic flow diagram of an alternative method for determining shape information SI of an ocular prosthesis according to an alternative embodiment. The method comprises a first step S1 of generating shape information of an existing patient-specific prosthesis, e.g. by 3D scanning said prosthesis. In a second step S2, said shape information are transformed into a uniform shape representation by performing the alignment and the identification of corresponding vertices as outlined above. These transformed shape information provide the shape information SI used for manufacturing the prosthesis 7.

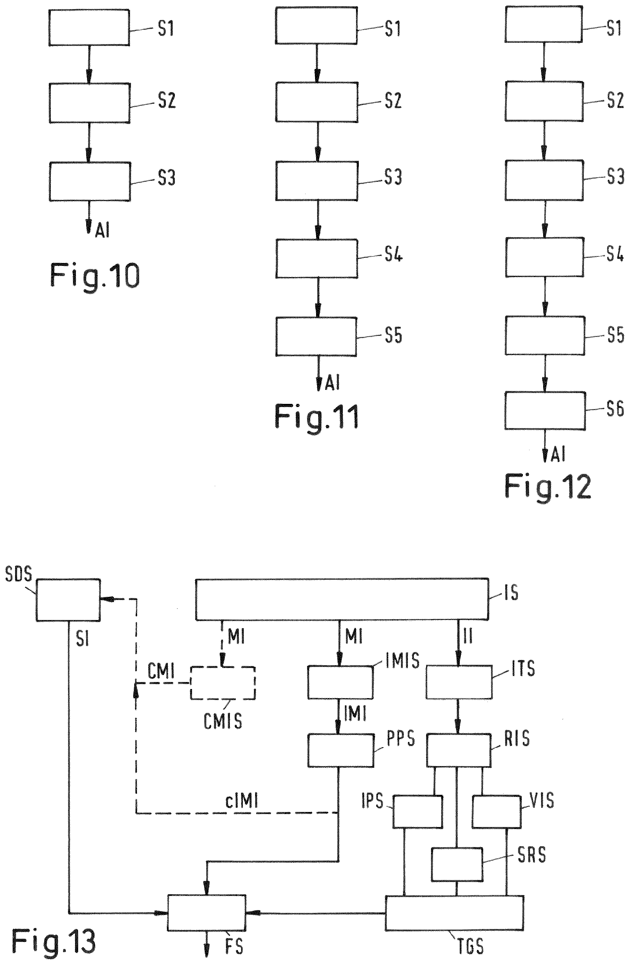

Fig. 10 shows a schematic flow diagram of determining appearance information AI of an ocular prosthesis 7 (see Fig. 1). The generation of appearance information AI comprises color imaging the patient's eye which can be the eye to be replace or the companion's eye. Such a two-dimensional image can be generated by an imaging device such as a camera in a first step S1. As a result of the first step S1, raw image data is provided. This data can provide image-device-dependent color information and can e.g. be RGB data. In a second step S2, this data providing image-device-dependent color information can be transformed into data providing image-device-independent color information such as CIEXYZ values. To determine the applicable transformation, a color characterization of the imaging device can be performed prior to the determination of appearance information AI shown in Fig. 10. This color characterization has been described before and can use the same physical imaging conditions as used for the eye appearance imaging. Further, the color characterization can be performed using the same or similar viewing conditions as a color characterization of a device or system 30 (see Fig. 15) used for the manufacturing of the ocular prosthesis 7 based on the appearance information Al, in particular considering the same predetermined color matching functions defining the observer as well as illuminant information. Physical imaging conditions can e.g. be characterized by predetermined imaging parameters such as a predetermined focus value, aperture value, working distance, exposure time etc. and lighting conditions, e.g. a predetermined intensity and (a) predetermined spectral power distribution. In a third step S3, an inhomogeneous illumination correction of the transformed data is performed. Alternatively, this correction can be performed before the transformation of the second step S2. Such a correction can involve generating a reference image of a target with a uniform reference color, in particular of a conformer with a reference color, prior to the determination of appearance information AI shown in Fig. 10. Then, pixel intensity correction values or correction parameters of a correction function can be determined such that the reference image is a uniform color image after the correction function has been applied or the pixel intensities have been corrected. The inhomogeneous illumination correction performed in the third step S3 is then performed by applying the correction function or by correcting the pixel intensities accordingly.

Further, at least one of a thermal noise correction, a specularity removal, a contrast enhancement and a vignetting correction can be performed in the third step S3 or before the transformation of the second step S2 is performed. In particular the thermal noise correction can be performed before the transformation of the second step S2, i.e. on the raw image. Further, an inpainting can be performed for the removed specularities. Such steps are preferable but optional.

The image being processed according to the previously described steps S1, S2, S3 can then be analysed as e.g. outlined in GB 2589698 A1 in order to fuse the shape information provided as outlined above and the appearance information AI provided by said image. This fusion can include mapping the texture information to the shape, e.g. by the well-known spherical or cone-like UV-mapping.

Fig. 11 shows a schematic flow diagram of determining appearance information AI of an ocular prosthesis 7 (see Fig. 1) according to another embodiment. In addition to the steps S1, S2, S3 of the embodiment shown in Fig. 10, the determination can further comprise a fourth step S4 of identifying at least one image region is identified in which a part of the eye is mapped. In particular, the determination can comprise a segmentation. More particular, the regions in which the iris 11 and sclera 8 (see e.g. Fig. 2) are depicted can be identified. However, further regions such as regions depicting veins, eyelids, eye lashes or the pupil can also be identified. Exemplary identification or segmentation methods have been outlined above, in particular segmentation methods being region growing methods such as a watershed algorithm. Such algorithms can involve the identification of seed points which has also been outlined above. In particular, the color image of the eye can be segmented in at least a pupil, iris, sclera, and tissue part using a watershed algorithm that operates on a median filtered image to label the sclera region, and a modified Daugman algorithm at multiple resolutions to determine the iris and pupil edges for subsequent labeling.

After different regions have been identified, these regions can be processed independently, in particular in a fifth step S5. It is e.g. possible to filter the sclera region by removing all sub regions associated in which veins or eye lashes are depicted, e.g. by identifying pixels with colors that have a red hue or are too dark. Then, a sclera base color and a sclera staining can be determined from the filtered region, e.g. by applying a k-means clustering on the colors. Then, a staining texture for the sclera can be determined based on said base color and staining information and can be applied to the sclera region. The base color can e.g. be determined form the brightest and the least chromatic colors of the clusters. It is then possible to generate Perlin noise, in particular for each color of the clusters, in order to generate the staining texture, wherein the Perlin noise is generated with predetermined parameters. In other words, the clustering provides the set of sclera colors from which the sclera base color is determined, e.g. by combining the colors based on the lightness. Then, Perlin noise textures are used to draw stains of each color of the sclera color set on the base color.

For the region depicting the iris, a lightness contrast can be increased. It is further possible to map the resulting iris region and its texture into cylindrical coordinate system, such that boundary to a sclera region and a boundary to pupil region form lines of constant height. For the region depicting the pupil, a color correction of the pixel color information can be performed such that the pupil is colored uniformly black.

Further, region-specific color information can be used to perform inpainting in a selected region.

Fig. 12 shows a schematic flow diagram of determining appearance information AI of an ocular prosthesis 7 (see Fig. 1) according to another embodiment. In addition to the steps S1, S2, S3, S4, S5 of the embodiment shown in Fig. 11, the method comprises a sixth step S6 introducing vein regions into the image, in particular into the sclera region. Prior to performing the sixth step S6, a veining recipe can be retrieved from a memory, wherein the veining recipe can e.g. be provided as a text file. Further, veining parameters can also be retrieved from a memory or can be determined based on the color calibrated image provided by the third step S3.

Procedurally generate a network of veins by growing veins in a predefined number, e.g. three, layers. Starting in the first layer with a fixed number of anatomically motivated seed points, each vein grows and branches into smaller vessels of the following layer. The veins and there growing and branching behavior are defined by vein recipes, these are stored in a veining recipe library. The vein recipes and the characteristics defined therein are modified by the veining parameters, e.g. such that the veins are grow thicker or thinner, or branch into more or fewer veins. Veins are modeled as a list of nodes in a 2D coordinate system. Each vein starts at some position, either a seed point or a branching point, and grows in a predetermined direction, e.g. towards the bottom, where the next nodes position and attributes, such as thickness and depth, is determined by a procedure considering the vein profile. The vein stops growing for example after a number of steps, after it becomes too thin or if it reaches the bottom that represents the limbus. Once the growth of all veins in a layer is simulated, for each vein a number of branching points is computed, then the vein growth process is started again with these veins. Once a certain vein layer has been grown, e.g. the third layer, the branching step is omitted and the veining generation is complete.

The veining network can then be rendered as B-Splines between these node points, e.g. using vein profiles that contain information of sampled cross-sections extracted from color calibrated images of eyes and are labeled with thickness and depth of the vein. These vein profiles can be determined in a semi-manual process offline and can be stored in a library/memory. Then, each vein can be generated or rendered with the closest matching vein profile in terms of thickness and depth, or a combination or interpolation of matching vein profiles. When merging the color information of the different layers the color information of overlapping veins can be combined by the depth, blending the colors such that the shallower vein dominates.

After the veining network has been rendered, it can be added to the sclera region, in particular to the recolored sclera region. In other words, the appearance of the sclera region can be replicated using the sclera labeled parts of the segmentation in the color image to create a texture that combines a base layer that replicates the sclera tissue itself and a veining layer that replicates the vessels in or above the sclera.

Fig. 13 shows a schematic flow diagram of determining shape and appearance information of an ocular prosthesis 7 (see e.g. Fig. 1). Shown is a shape determination step SDS which can e.g. comprise the steps S1, S2, S3, S4 shown in Fig. 4 or Fig. 9 or subset thereof. The shape determination step SDS provides shape information SI which are used for manufacturing a prosthesis 7 (see Fig. 1).

Further shown is an imaging step IS by which a two-dimensional image II of the patient's eye is generated, e.g. using a suitable imaging device. Further, the imaging step IS can comprise the generation of socket surface information by scanning the eye socket 2, in particular by using a shape scanning device 25 (see Fig. 14) which corresponds to step S1 of Fig. 4.

It is possible but not mandatory that in this imaging step IS, mesh information MI are generated, e.g. by the used scanning device 25. These mesh information MI can be three-dimensional information of the imaged eye. Based on said mesh information, cornea mesh information CMI are determined in a cornea mesh information generation step CMIS which encode a three dimensional representation of the cornea. Also, iris mesh information IMI are determined in an iris mesh information generation step IMIS which encode a three dimensional representation of the iris. In these steps CMIS, IMIS, the voxels belonging to the cornea or iris can be determined in a selected coordinate system, in particular in a coordinate system which is co-registered to the image coordinate system of the generated 2D image II and/or co-registered to the scan data provided by the scanning device 25.

Further, an image transformation step ITS is performed by which image data providing image-device-dependent color information can be transformed into data providing image-device-independent color information. This image transformation step ITS can correspond to the second step S2 of Fig. 10. Further, a region identification step RIS can be performed in order to identify the sclera, the iris and the pupil region in the transformed image.

Based on the transformed image, a vein introduction step VIS is performed which can correspond to the sixth step S6 shown in Fig. 12. The vein introduction step can be performed for the identified sclera region only. It is further possible to assign depth information to the generated veins, e.g. in form of a displacement map.

In a sclera recoloring step SRS is performed for the sclera region. This step can correspond to the sclera-related parts of the fifth step S5 shown in Fig. 11. Further, an iris processing step IPS is performed, in particular to increase a lightness contrast. This step can correspond to the iris-related parts of the fifth step S5 shown in Fig. 11. As this step S5, the iris processing step IPS can comprise a color correction of the pupil pixel color information in order to ensure that the pupil is colored uniformly black.

Further shown is a pupil processing step PPS which is performed based on the iris mesh information IMI. An exemplary iris mesh 31 is shown in Fig. 7C aligned in the reference coordinate system with the axes x, y, z.

In this step, the iris mesh information is adjusted such that the iris edge or border which encloses the pupil region is round or essentially round and arranged in a non-curved plane, in particular perpendicular to the vertical axis. The corrected iris mesh information cIMI as well as the cornea mesh information CMI is feed into the shape determination step SDS, in particular in order to adapt the determined shape of the ocular prosthesis 7 according to mesh information. Such an adaption can e.g. comprise to adjust the position of the vertices being arranged on the circle of the limbus such that the updated circle provided by the changed positions is adapted in size and/or form to the iris mesh. The mesh generation steps CMIS, IMIS as well as the steps based thereon are, however, optional steps.

In a texture generation step TGS, a texture comprising, if applicable, depth or displacement information, is generated based on the output of the region identification step RIS, the iris processing step IPS, the sclera recoloring step SRS and the vein introduction step VIS. Suitable texture generation algorithms are known to the skilled person.

It is possible to map the segmented iris texture into a cylindrical coordinate system such that a boundary to sclera and a boundary to pupil form lines of constant height and then unwrap into texture.

In a fusing step FS, the generated textures (for the different regions) are mapped on the previously generated shape or shape objects using e.g. a spherical or cone-like UV-mapping.

Combining the iris and sclera texture as well as geometry at the seam of the limbus should blur the transition at the limbus. This can be achieved or enhanced by applying filter algorithms such as Gaussian Filters to filter the color information at and close to the transition. Further, transparency information of the texture can be adjusted to increase the light transport in the manufacturing process. Alternatively, creating overlaps in the geometry is possible.

The combined UV mapped geometries can be stored in a .obj file, the textures can be stored as a .png image for color and .tiff images for displacement or clear coating.

Fig. 14 shows a schematic block diagram of a system 24 for determining shape and appearance information SI, AI of an ocular prosthesis 7 for a patient (see Fig. 1). The system 24 comprises a shape scanning device 25 which can e.g. be an OCT scanning device. Further, the system 24 comprises an appearance imaging device for imaging a patient's eye which can e.g. be a colour imaging device such as a camera. Further, the system 24 can comprise an evaluation unit 27 such as a computing device, wherein the evaluation unit/computing device can comprise or be provided by at least one microcontroller or integrated circuit. Means for generating shape information SI for the ocular prosthesis 7 can comprise the shape scanning device 25 as well as the evaluation unit 27, wherein the evaluation unit 27 determines the shape information SI based on the scan data provided by the shape scanning device 25. Means for generating appearance information AI for the ocular prosthesis 7 can comprise the appearance imaging device 26 as well as the evaluation unit 27 (or a further evaluation unit which is not shown), wherein the evaluation unit 27 (or the further evaluation unit) determines the appearance information AI based on the image data provided by the appearance imaging device 26. The evaluation unit 27 (or a further evaluation unit) can also provide means for fusing the shape and the appearance information SI, Al. The system 24 can further comprise at least one memory unit (not shown) for storing e.g. a correction value for image processing and/or information of a vein recipe. The system 24 is in particular configured to perform one of the methods outlined in this invention.

Fig 15 shows a schematic block diagram of a system 30 for manufacturing an ocular prosthesis 7 (see Fig. 1) for a patient. The system 30 comprises a system 24 for determining shape and appearance information SI, AI of an ocular prosthesis 7 as outlined above. Further, the system 30 comprises means for manufacturing the ocular prosthesis 7 according to the shape and appearance information provided by the system 24, in particular the evaluation unit 27 of said system 24. Means for manufacturing can be provided by a printing system, wherein the printing system comprises a 3D printing device 29 and at least one control unit 28, wherein the control unit 28 is configured to generating a control signal for the printing device 29 based on the shape and appearance information. The printing system can be a printing system for producing the prosthesis 7. The control unit 28 and the printing device 29 can be provided by separate devices or by a common device. The evaluation unit 27 and the control unit 28 can be connected by a wired or wireless data connection, wherein the data encoding shape and appearance information SI, AI generated by the evaluation unit 27 is transmitted to the control unit 28 via the data connection. The control unit 28 can have an interface to receive such data. It is, however, also possible that the data encoding shape and appearance information SI, AI is stored in a memory device, e.g. a portable device, and said control unit 28 accesses the data stored in this memory device in order to generate the control signals.

The control unit 28 and the printing device 29 can be connected by a wired or wireless data connection, wherein the control data generated by the control unit 28 is transmitted to the printing device 29 via the data connection. The printing device 29 can have an interface to receive the control data. The printing device can comprise at least one means for printing a printing material, e.g. one or more print heads. The 3D printing device 29 can also be referred to as additive manufacturing device.

Fig. 16 shows a schematic flow diagram of a system for manufacturing an ocular prosthesis 7 for a patient. The method comprises a shape determining step SDS for determining the (resulting) shape information SI of the ocular prosthesis 7. Further, the method comprises an appearance determination step ADS for determining the appearance information Al. Further, the method comprises a manufacturing or printing step PS for manufacturing the ocular prosthesis 7 according to the shape and appearance information SI, Al.

## Claims

1. A method of determining shape and appearance information of an ocular prosthesis (7) for a patient, in particular for the manufacturing of the ocular prosthesis (7), comprising the steps of:
- generating shape information for the ocular prosthesis (7),
- determining the shape of the ocular prosthesis (7) depending on said shape information,
- generating appearance information for the ocular prosthesis (7) by capturing an image of a patient's eye,
- fusing the shape and the appearance information,
**characterized in that**
the determination of the shape of the ocular prosthesis (7) comprises
- determining the shape based on a shape model being determined based on the shapes of existing prostheses or
- determining the shape by generating shape information of an existing patient-specific prosthesis and transforming it into a uniform shape representation, and/or
the generation of appearance information comprises performing at least one of
- an inhomogeneous illumination correction and
- a color characterization being performed using the same or similar viewing conditions as a color characterization of a device used for the manufacturing of the ocular prosthesis based on the appearance information.

2. The method according to claim 1, **characterized in that** the shape of the ocular prosthesis (7) is determined based on at least one reference shape for the ocular prosthesis (7).

3. The method according to one of the preceding claims, **characterized in that** the reference shape is selected based on at least one existing conformer (19) which is selected from a set of multiple conformers.

4. The method according to one of the preceding claims, **characterized in that** socket surface shape information (23) and the shape model are aligned in a common reference coordinate system, wherein alignment parameters are determined based on at least one optically detectable landmark of the conformer (19), wherein the optically detectable landmark is detected in an image providing/encoding the socket surface shape information (23).

5. The method according to one of the preceding claims, **characterized in that** the shape of the ocular prosthesis (7) is determined such that a difference metric is minimized, wherein the difference metric is determined as a function of a deviation between the shape of the ocular prosthesis (7) and the socket surface shape (23).

6. The method according to one of the preceding claims, **characterized in that** the difference metric is further determined depending on a second deviation between the shape of the ocular prosthesis and a reference shape provided by/according to the shape model.

7. The method according to claim 5 to 6, **characterized in that** minimizing the difference metric is performed by varying at least one model parameter, wherein the shape of the ocular prosthesis (7) is determined as the shape provided by the shape model using the at least one parameter which minimizes the difference metric.

8. The method according to one of the claims 5 to 7, **characterized in that** socket surface shape information (23) and the shape model are aligned in a common reference coordinate system, wherein minimizing the difference metric is performed by varying at least one alignment parameter.

9. The method according to one of the preceding claims, **characterized in that** a transparent layer is added to a least a section of the surface of the determined shape of the ocular prosthesis (7) and/or the determined shape of the ocular prosthesis (7) is adapted according to mesh information.

10. The method according to one of the preceding claims, **characterized in that** the generation of appearance information further involves at least one of a thermal noise correction, a specularity removal, a vignetting correction, an inpainting for providing image information for an image area which is identified as an image area not mapping a part of the eye to be reproduced, a contrast enhancement, a filtering of an identified region, a reshaping of an identified region and a recoloring of an identified region.

11. The method according to one of the preceding claims, **characterized in that** at least one image region is identified in which a part of the eye is mapped.

12. The method according to one of the preceding claims, **characterized in that** a vein generation is performed for introducing vein regions into the image.

13. A computer program product with a computer program, wherein the computer program comprises software means for the execution of one, multiple or all steps of the method of determining shape and appearance information of an ocular prosthesis (7) for a patient according to one of the claims 1 to 12 if the computer program is executed by or in an automation system.

14. A system for determining shape and appearance information of an ocular prosthesis (7) for a patient, in particular for the manufacturing of the ocular prosthesis, the system (24) comprising at least one means for generating shape information for the ocular prosthesis (7), at least one means for determining the shape of the ocular prosthesis (7) depending on the shape information, at least one means for generating appearance information for the ocular prosthesis (7) by capturing an image of a patient's eye and at least one means for fusing the shape and the appearance information,
**characterized in that**
the determination of the shape of the ocular prosthesis (7) comprises
- determining the shape based on a shape model being determined based on the shapes of existing prostheses or
- determining the shape by generating shape information of an existing patient-specific prosthesis and transforming it into a uniform shape representation, and/or
the generation of appearance information comprises performing at least one of
- an inhomogeneous illumination correction and
- a color characterization being performed using the same or similar viewing conditions as a color characterization of a device used for the manufacturing of the ocular prosthesis based on the appearance information.

15. A conformer for the use in a method of determining shape and appearance information of an ocular prosthesis (7) for a patient according to one of the claims 1 to 12, the conformer (19) having or providing at least one optically detectable landmark, wherein the optically detectable landmark is detectable in an image providing/encoding the socket surface information.
